# EUROPEAN PATENT APPLICATION

(11) **EP 3 358 828 A1**
(43) Date of publication of application: **08.08.2018**
(21) Application number: 16851453.7
(22) Date of filing: 26.09.2016
(51) Int. Cl.: H04N 5/374, A61B 1/04, G02B 23/24, H01L 27/14, H01L 27/146, H04N 9/07

(54) **IMAGE PICKUP ELEMENT AND ENDOSCOPE**

(30) Priority: 02.10.2015 JP 2015197006
(71) Applicant: Olympus Corporation, Hachioji-shi Tokyo 192-8507 (JP)
(72) Inventor: TANAKA, Takanori, Hachioji-shi Tokyo 192-8507 (JP); AKAHANE, Nana, Hachioji-shi Tokyo 192-8507 (JP); ADACHI, Satoru, Hachioji-shi Tokyo 192-8507 (JP)
(74) Representative: Gunzelmann, Rainer
(86) International application number: PCT/JP2016/078287
(87) International publication number: WO 2017/057282

(57) **Abstract**

The present invention provides an image sensor that can prevent variations from occurring in image signals from pixels in which filters of the same color are arranged on the light receiving surface. That is, the image sensor includes a timing generator 25 that, based on the type of the filter disposed on the light receiving surface of the photoelectric conversion element to which the imaging signal is output, causes a switching unit 245 to switch the transfer destination of the imaging signal transferred from a first horizontal transfer line 259 and a third horizontal transfer line 261 to either a first output amplifier unit 311 or a second output amplifier unit 312, and in the case of outputting the imaging signal from the unit pixel 230 for one row to the plurality of sample-and-hold units from the plurality of pixel units G1 and G2, controls the vertical scanning unit 241 so as to output the imaging signal in a predetermined number of times during one horizontal scanning period, and at each time of output, output the imaging signal from the photoelectric conversion element in which different kinds of filters are arranged on the light receiving surface from each pixel unit.

## Description

### Field

The present invention relates to an image sensor and an endoscope for capturing images of a subject to generate image data of the subject.

### Background

Recently, there has known a technique relating to complementary metal oxide semiconductor (CMOS) image sensors for transferring an image signal by sharing two pixels adjacent in the row direction by one vertical signal line (refer to Patent Literature 1). In this technique, reading of image signals for one line is performed in two steps of reading of image signals of odd number columns (for example, R pixels) and reading of image signals of even number columns (for example, G pixels).

### Citation List

### Patent Literature

Patent Literature 1: Japanese Patent No. 5596888

### Summary

### Technical Problem

As the number of pixels and the speed of the image sensor are increased, it is necessary to increase the speed of a signal processing circuit (e.g., an output amplifier unit and an A/D converter) for processing image signals read out by horizontal scanning. Therefore, it may be possible to provide a plurality of signal processing circuits in the conventional image sensor to increase the speed by parallel processing.

However, if the plurality of signal processing circuits is provided in Patent Literature 1, imaging signals are read out from pixels having filters of the same color arranged on the light receiving surface during each reading. Therefore, even pixels having filters of the same color arranged on the light receiving surface output image signals via different signal processing circuits. Hence, variation in the image signals occurs due to a potential fluctuation between channels in the signal processing circuits or signal transmission paths.

The present invention has been made in view of the foregoing, and an object of the invention is to provide an image sensor capable of avoiding the variation in the image signals from pixels having filters of the same color arranged on the light receiving surface.

### Solution to Problem

In order to solve the above described problem and achieve the object, an image sensor according to the invention includes: a light receiving unit including: a plurality of unit pixels arranged in a two-dimensional matrix form, each of the plurality of unit pixels having a plurality of photoelectric converters for converting received light into imaging signals and outputting the imaging signals; and filters having different transmission spectra and disposed on light receiving surfaces of the plurality of photoelectric converters adjacent in a row direction; a plurality of first transfer lines each sharing a predetermined number of pixels in the row direction and configured to transfer the imaging signals; a constant current source provided for each of the plurality of first transfer lines and configured to transfer the imaging signals output from the plurality of unit pixels to each of the plurality of first transfer lines; a plurality of sample-and-hold units provided for the plurality of first transfer lines and configured to sample the imaging signals; a plurality of second transfer line configured to transfer the imaging signals sampled by the plurality of sample-and-hold units; a plurality of signal processing units provided corresponding to the number of the plurality of second transfer lines, and configured to perform signal processing on the imaging signals transferred from the plurality of second transfer lines and output the imaging signals to outside; a switching unit provided between the plurality of second transfer lines and the plurality of signal processing units and configured to switch a connection between the plurality of second transfer lines and the plurality of signal processing units; a vertical scanning unit configured to output the imaging signals from the plurality of unit pixels to the plurality of sample-and-hold units via the plurality of first transfer lines; and a control unit configured to cause the vertical scanning unit to: switch between the plurality of signal processing units with respect to transfer destination of the imaging signals transferred from the plurality of second transfer lines to the switching unit, based on types of the filters disposed on the light receiving surfaces of the plurality of photoelectric converters from which the imaging signals are output; and output the imaging signals from a single row of the plurality of unit pixels to the plurality of sample-and-hold units, in a predetermined number of times during one horizontal scanning period by dividing the plurality of unit pixels into multiple pixel units each time the imaging signals are output so as to output the imaging signals from the plurality of photoelectric converters having the light receiving surfaces on which the filters of different types are disposed in each of the multiple pixel units.

In the image sensor according to the invention, the control unit is configured to cause the switching unit to switch the transfer destination of the imaging signals transferred from the plurality of second transfer lines so as to transfer the imaging signals output from the plurality of photoelectric converters having the light receiving surfaces on which the filters of a same type are disposed, to a same signal processing unit of the plurality of signal processing units.

In the image sensor according to the invention, each of the plurality of unit pixels includes: a charge voltage converter; a plurality of charge transfer units configured to transfer charges from the plurality of photoelectric converters to the charge voltage converter; and an output unit configured to output the imaging signals after voltage conversion by the charge voltage converter. The number of the plurality of charge transfer units in each row of the plurality of unit pixels is two. Eeach of the two charge transfer units is connected to a first signal line or a second signal line to input a command signal for outputting the imaging signals at different points in time. The number of the multiple pixel units is two. A type of one of the filters disposed on one of the plurality of photoelectric converters to output the imaging signals from a first one of the two charge transfer units connected to the first signal line in a first one of the two pixel units is different from a type of another one of the filters disposed on another one of the plurality of photoelectric converters to output the imaging signals from the first one of the two charge transfer units connected to the first signal line in a second one of the two pixel units. A type of one of the filters disposed on one of the plurality of photoelectric converters to output the imaging signals from a second one of the two charge transfer units connected to the second signal line in the first one of the two pixel units is different from a type of another one of the filters disposed on another one of the plurality of photoelectric converters to output the imaging signals from the second one of the two charge transfer units connected to the second signal line in the second one of the two pixel units.

In the image sensor according to the invention, each of the filters disposed on the light receiving surfaces of the plurality of photoelectric converters is one of a red filter, a green filter, and a blue filter. The number of the plurality of signal processing units is two. One of the two signal processing units is configured to perform the signal processing only on the imaging signals output from a photoelectric converter on which the green filter is disposed, among the plurality of photoelectric converters.

An endoscope according to the invention includes the image sensor on a distal end of an insertion portion configured to be inserted into a subject.

### Advantageous Effects of Invention

According to the present invention, it is possible to avoid variation in image signals from pixels having filters of the same color arranged on a light receiving surface.

### Brief Description of Drawings

FIG. 1 is a schematic diagram schematically illustrating the overall configuration of an endoscope system according to a first embodiment of the present invention.
FIG. 2 is a block diagram illustrating functions of a main part of the endoscope system illustrated in FIG. 1.
FIG. 3 is a circuit diagram illustrating a configuration of a first chip illustrated in FIG. 2.
FIG. 4 is a timing chart illustrating drive timing of an imaging unit according to the first embodiment of the present invention.
FIG. 5A is a circuit diagram of a CDS circuit according to a modification of the first embodiment of the present invention.
FIG. 5B is a circuit diagram illustrating a configuration of a first chip according to a second embodiment of the present invention.
FIG. 6 is a timing chart illustrating drive timing of the imaging unit according to the second embodiment of the present invention.
FIG. 7 is a circuit diagram illustrating a configuration of a first chip according to a modification of the second embodiment of the present invention.
FIG. 8 is a circuit diagram illustrating a configuration of a first chip according to a third embodiment of the present invention.
FIG. 9 is a timing chart illustrating drive timing of the imaging unit according to the third embodiment of the present invention.
FIG. 10 is a circuit diagram illustrating a configuration of a first chip according to a fourth embodiment of the present invention.

### Description of Embodiments

Hereinafter, as modes for carrying out the invention (hereinafter referred to as "embodiment(s)"), reference will be made to an endoscope system including an endoscope in which an image sensor is provided at a distal end of an insertion portion configured to be inserted into a subject. The present invention is not limited by the embodiments. The same reference signs are used to designate the same elements throughout the drawings. The drawings are schematic, the relationship between the thickness and the width of each member, the ratio of each member, etc. are different from the reality. Differences between dimensions and ratios of each other are also included in the drawings.

### (First Embodiment)

### [Configuration of Endoscope System]

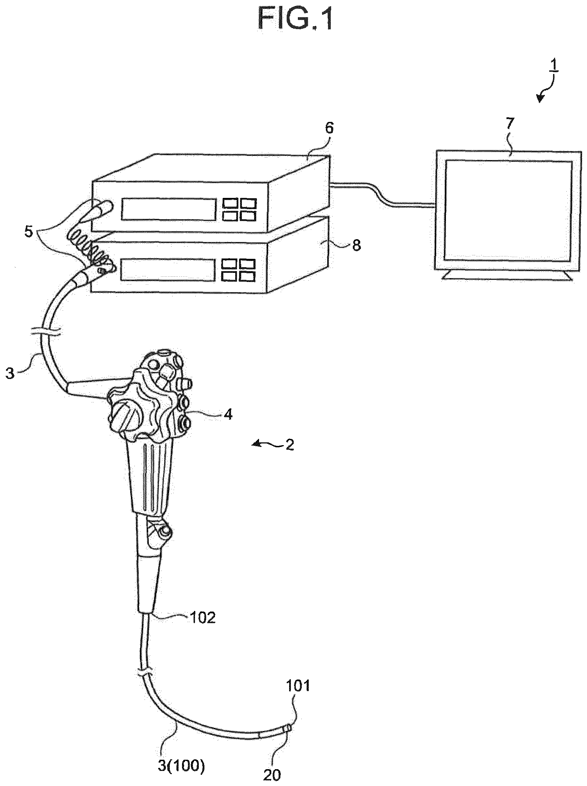

FIG. 1 is a schematic diagram schematically illustrating the overall configuration of an endoscope system according to a first embodiment of the present invention. An endoscope system 1 illustrated in FIG. 1 includes an endoscope 2, a transmission cable 3, a connector unit 5, a processor 6 (processing device), a display device 7, and a light source device 8.

The endoscope 2 images the inside of the body of the subject by inserting an insertion portion 100 which is a part of the transmission cable 3 into a body cavity of the subject, and outputs the imaging signal (image data) to the processor 6. Further, the endoscope 2 is one end of the transmission cable 3, an imaging unit 20 (imaging device) that images an in-vivo image is provided on a distal end 101 side of the insertion portion 100 to be inserted into the body cavity of the subject. An operating unit 4 for receiving various operations on the endoscope 2 is provided on a proximal end 102 side of the insertion portion 100. The imaging signal of the image imaged by the imaging unit 20 passes through the transmission cable 3 having a length of several meters, for example, and is output to the connector unit 5.

The transmission cable 3 connects the endoscope 2 and the connector unit 5 and connects the endoscope 2 and the light source device 8. Further, the transmission cable 3 propagates the imaging signal generated by the imaging unit 20 to the connector unit 5. The transmission cable 3 is configured using a cable, an optical fiber, or the like.

The connector unit 5 is connected to the endoscope 2, the processor 6, and the light source device 8, performs predetermined signal processing on imaging signals outputted from the connected endoscope 2, and converts an analog imaging signal into a digital imaging signal (A/D conversion) and outputs the digital imaging signal to the processor 6.

The processor 6 is configured to perform predetermined image processing on an imaging signal input from the connector unit 5 and outputs the imaging signal to the display device 7. In addition, the processor 6 is configured to perform overall control of the endoscope system 1. For example, the processor 6 is configured to switch the illumination light emitted from the light source device 8 and to switch between the imaging modes of the endoscope 2.

The display device 7 is configured to display images corresponding to the imaging signal after the image processing by the processor 6. Further, the display device 7 displays various kinds of information on the endoscope system 1. The display device 7 is configured using a display panel such as liquid crystal or organic EL (Electro Luminescence) or the like.

The light source device 8 is configured to irradiate the subject with the illumination light from the distal end 101 of the insertion portion 100 of the endoscope 2 via the connector unit 5 and the transmission cable 3. The light source device 8 includes a white light emitting diode (LED) for emitting white light and an LED for emitting special light of narrow band light having a narrower wavelength band than a wavelength band of white light. Under the control of the processor 6, the light source device 8 is configured to irradiate the subject with white light or narrow band light via the endoscope 2.

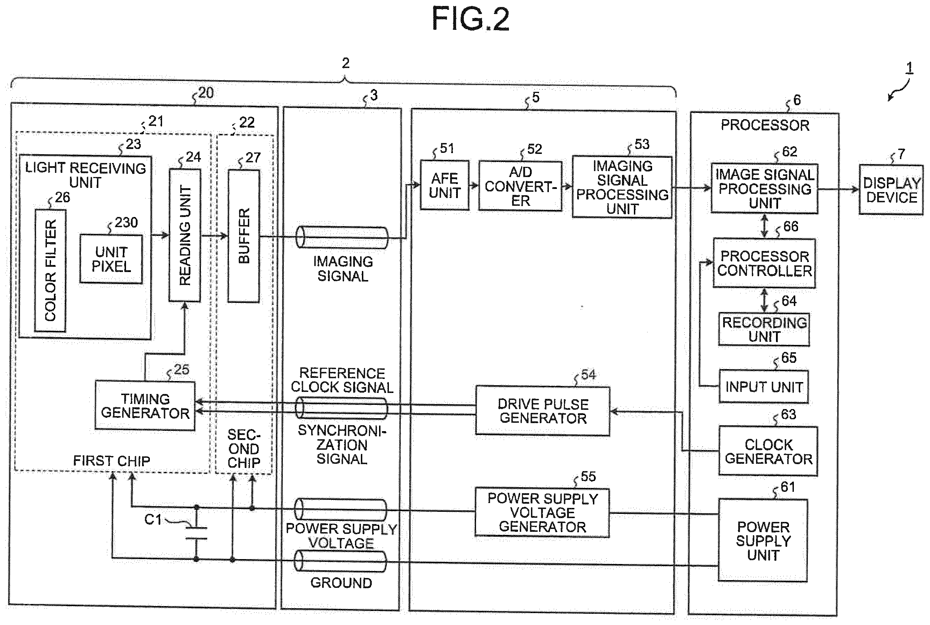

FIG. 2 is a block diagram illustrating functions of a main part of the endoscope system 1. With reference to FIG. 2, the details of each part configuration of the endoscope system 1 and the path of the electric signal in the endoscope system 1 will be described.

### [Configuration of Endoscope]

First, the configuration of the endoscope 2 will be described. The endoscope 2 illustrated in FIG. 2 includes the imaging unit 20, the transmission cable 3, and the connector unit 5.

The imaging unit 20 includes a first chip 21 (image sensor) and a second chip 22. The imaging unit 20 receives the power supply voltage VDD generated by a power supply voltage generator 55 of the connector unit 5 described below via the transmission cable 3, together with the ground GND. A power supply stabilizing capacitor C1 is provided between the power supply voltage VDD supplied to the imaging unit 20 and the ground GND.

The first chip 21 includes a light receiving unit 23 which is arranged in a two-dimensional matrix and has a plurality of unit pixels 230 which receive light from the outside and generate and output an image signal corresponding to the amount of received light, a reading unit 24 that reads out the imaging signal photoelectrically converted by each of the plurality of unit pixels 230 in the light receiving unit 23, a timing generator 25 that generates a timing signal based on a reference clock signal and a synchronization signal input from the connector unit 5 and outputs the timing signal to the reading unit 24, and a color filter 26 disposed on each light receiving surface of the plurality of unit pixels 230. A more detailed configuration of the light receiving unit 23, the reading unit 24, and the timing generator 25 in the first chip 21 will be described later.

The color filter 26 is realized by using a color filter of Bayer array configured by a red filter (hereinafter referred to as "R filter") having a maximum value of a transmission spectrum in a red wavelength band, a green filter (hereinafter referred to as "G filter") having a maximum value of a transmission spectrum in a green wavelength band, and a blue filter (hereinafter referred to as "B filter") having a maximum value of a transmission spectrum in a blue wavelength band. In the color filter 26, each of the R filter, the G filter, the B filter, and the G filter is disposed on each unit pixel 230. Specifically, in the color filter 26, the R filter and the G filter are alternately arranged in this order in the odd-numbered rows of the light receiving unit 23, and the G filter and the B filter are alternately arranged in this order in the even-numbered rows of the light receiving unit 23. In the following description, a photoelectric conversion element having a light receiving surface on which the R filter is disposed is referred to as a R pixel, a photoelectric conversion element on which the G filter is disposed is referred to as a G pixel, and a photoelectric conversion element on which the B filter is disposed is referred to as a B pixel.

The second chip 22 has a buffer 27 for amplifying an imaging signal output from each of the plurality of unit pixels 230 in the first chip 21 and outputting the imaging signal to the transmission cable 3. Note that the combination of circuits arranged in the first chip 21 and the second chip 22 can be appropriately changed. For example, the timing generator 25 arranged in the first chip 21 may be arranged in the second chip 22.

The connector unit 5 includes an analog front end unit 51 (hereinafter referred to as "AFE unit 51"), an A/D converter 52, an imaging signal processing unit 53, a drive pulse generator 54, and a power supply voltage generator 55.

The AFE unit 51 receives the imaging signal propagated from the imaging unit 20, performs impedance matching using a passive element such as a resistor, takes out an AC component by using a capacitor, and determines an operating point by a voltage dividing resistor. Thereafter, the AFE unit 51 corrects the imaging signal (analog signal) and outputs the imaging signal to the A/D converter 52.

The A/D converter 52 converts the analog imaging signal input from the AFE unit 51 into a digital imaging signal and outputs the digital imaging signal to the imaging signal processing unit 53.

The imaging signal processing unit 53 is constituted by, for example, a Field Programmable Gate Array (FPGA), performs processing such as noise removal and format conversion processing on the digital imaging signal input from the A/D converter 52, and outputs the resultant signal to the processor 6.

Based on a reference clock signal (for example, a clock signal of 27 MHz) that is supplied from the processor 6 and serves as a reference of the operation of each element of the endoscope 2, the drive pulse generator 54 generates a synchronization signal indicating the start position of each frame and outputs the synchronization signal together with the reference clock signal to the timing generator 25 of the imaging unit 20 via the transmission cable 3. Here, the synchronization signal generated by the drive pulse generator 54 includes a horizontal synchronization signal and a vertical synchronization signal.

The power supply voltage generator 55 generates a power supply voltage necessary for driving the first chip 21 and the second chip 22 from the power supplied from the processor 6 and outputs the power supply voltage to the first chip 21 and the second chip 22. The power supply voltage generator 55 generates a power supply voltage necessary for driving the first chip 21 and the second chip 22 by using a regulator or the like.

### [Configuration of Processor]

Next, the configuration of the processor 6 will be described.

The processor 6 is a control device that totally controls the entire endoscope system 1. The processor 6 includes a power supply unit 61, an image signal processing unit 62, a clock generator 63, a recording unit 64, an input unit 65, and a processor controller 66.

The power supply unit 61 generates the power supply voltage VDD and supplies the generated power supply voltage VDD to the power supply voltage generator 55 of the connector unit 5 together with the ground (GND).

The image signal processing unit 62 performs, on the digital imaging signal subjected to the signal processing by the imaging signal processing unit 53, image processing such as a synchronization process, a white balance (WB) adjustment process, a gain adjustment process, a gamma correction process, a digital analog (D/A) conversion process, a format conversion process, and converts the digital imaging signal into an image signal, and outputs the image signal to the display device 7.

The clock generator 63 generates a reference clock signal which serves as a reference for the operation of each element of the endoscope system 1 and outputs the reference clock signal to the drive pulse generator 54.

The recording unit 64 records various kinds of information on the endoscope system 1, data under processing, and the like. The recording unit 64 is configured by using a recording medium of a flash memory or a RAM (Random Access Memory).

The input unit 65 receives inputs of various operations related to the endoscope system 1. For example, the input unit 65 receives input of a command signal for switching the type of illumination light emitted from the light source device 8. The input unit 65 is configured using, for example, a cross switch, a push button, or the like.

The processor controller 66 totally controls each unit constituting the endoscope system 1. The processor controller 66 is configured using a CPU (Central Processing Unit) or the like. The processor controller 66 switches the illumination light emitted from the light source device 8 in accordance with the command signal input from the input unit 65.

### [Configuration of First Chip]

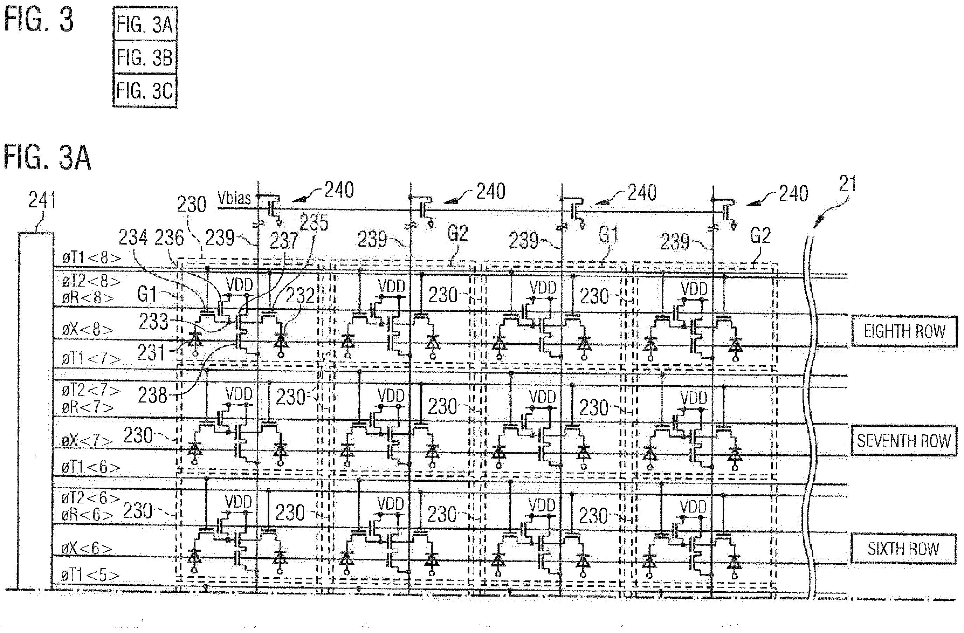

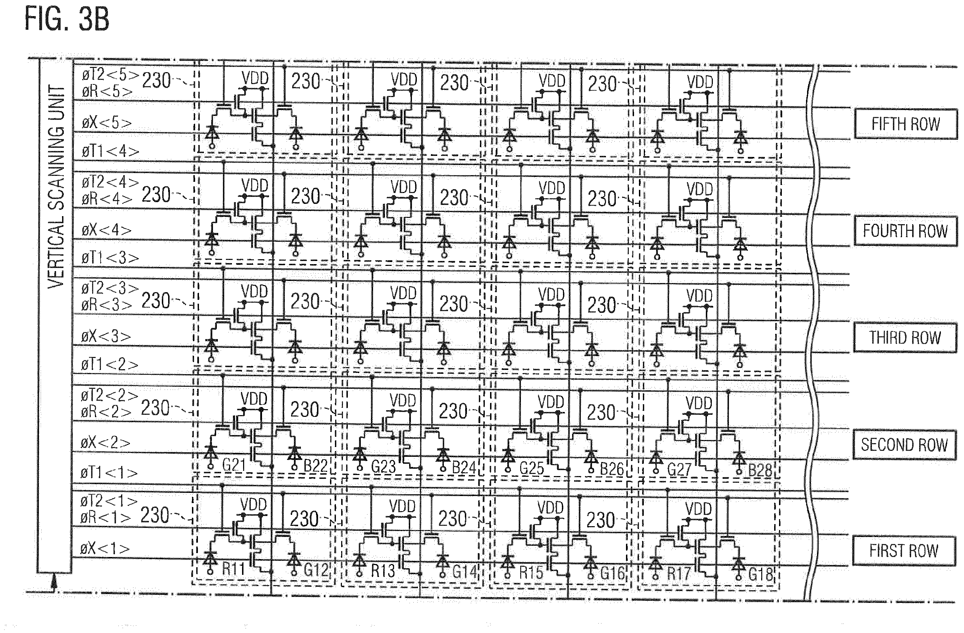

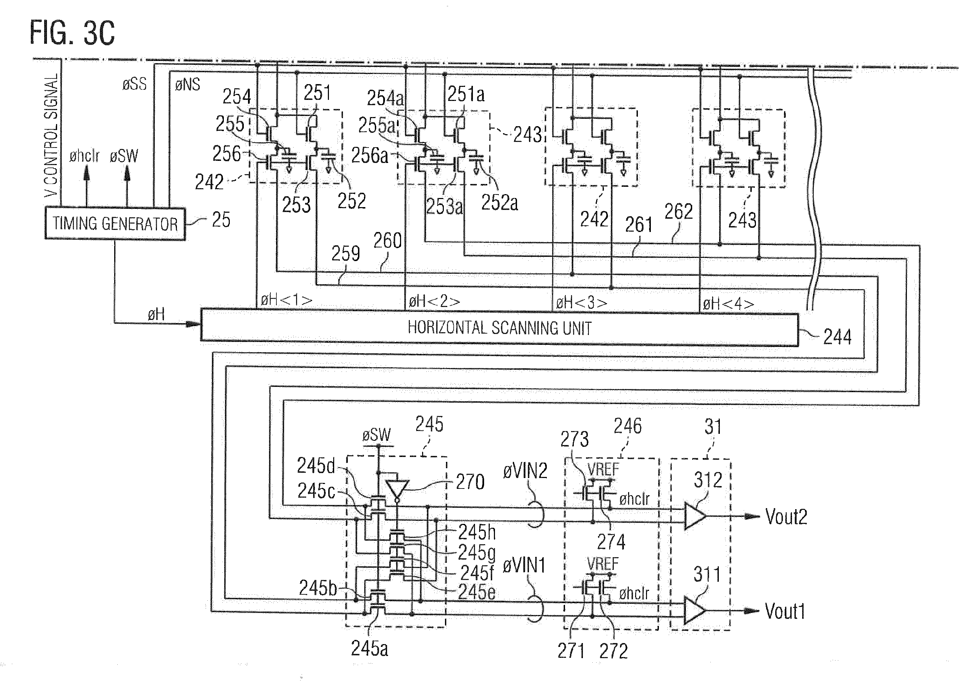

Next, the detailed configuration of the above-described first chip 21 will be described. FIG. 3 is a circuit diagram illustrating a configuration of the first chip 21 illustrated in FIG. 2. In FIG. 3, a coordinate of the photoelectric conversion element in which the R filter, the G filter, and the B filter are arranged is expressed as Rxy. For example, in FIG. 3, one row and one column of the photoelectric conversion element in which the R filter is arranged is R11, and one row and two columns of the photoelectric conversion element in which the G filter is arranged are expressed as G12.

As illustrated in FIG. 3, the first chip 21 includes a timing generator 25, an output unit 31, a constant current source 240, a vertical scanning unit 241 (row selection circuit), a first sample-and-hold unit 242, a second sample-and-hold unit 243, a horizontal scanning unit 244 (column selecting circuit), a switching unit 245, and a horizontal reset unit 246.

Based on the reference clock signal and the synchronization signal, the timing generator 25 generates various drive pulses (V control signals, φhclr, φNS, φSS, φH and φSW), and outputs the various drive pulses to the vertical scanning unit 241, the first sample-and-hold unit 242, the second sample-and-hold unit 243, the horizontal scanning unit 244, the switching unit 245, and the horizontal reset unit 246 which will be described later. In the first embodiment, the timing generator 25 functions as a control unit. That is, based on the type of the filter disposed on the light receiving surface of the photoelectric conversion element to which the imaging signal is output, the timing generator 25 causes the switching unit 245 to switch the transfer destination of the imaging signal transferred from a second horizontal transfer line 260 and a fourth horizontal transfer line 262 to either a first output amplifier unit 311 or a second output amplifier unit 312, and in the case of outputting the imaging signal from the unit pixel 230 for one row to the plurality of sample-and-hold units from the plurality of pixel units G1 and G2, controls the vertical scanning unit 241 so as to output the imaging signal in a predetermined number of times during one horizontal scanning period, divide the unit pixel 230 into a plurality of pixel units at each time of output, and output the imaging signal from the photoelectric conversion element in which different kinds of filters are arranged on the light receiving surface from each pixel unit.

One end of the constant current source 240 is connected to the ground GND, the other end is connected to a vertical transfer line 239, and a signal line to which the reference voltage Vbias is input is connected to the gate. The constant current source 240 is provided in each vertical transfer line 239 (first transfer line).

Based on the V control signals (φX, φR, φT1, φT2, etc.), by applying, to the selected row <M>(M = 1, 2, ..., m) of the light receiving unit 23 , each of a row selection pulse φX<M> , a drive pulse φ R<M> , a drive pulse φT1<M> and a drive pulse φT2<M>, and by driving each unit pixel 230 of the light receiving unit 23 with the constant current source 240 connected to the vertical transfer line 239, the vertical scanning unit 241 transfers the imaging signal and the noise signal at the time of pixel reset by the vertical transfer line 239, and outputs each of the noise signal and the imaging signal to the first sample-and-hold unit 242 or the second sample-and-hold unit 243. In the first embodiment, an example in which two photoelectric conversion elements are shared by one vertical transfer line 239 and an imaging signal is read out will be described.

The first sample-and-hold unit 242 (sample-and-hold circuit) samples the noise signal at the time of pixel reset in each unit pixel 230 which outputs the imaging signal via the vertical transfer line 239 of the odd number column, and outputs the sampled noise signal to the output unit 31. Furthermore, the first sample-and-hold unit 242 samples the imaging signal photoelectrically converted by each unit pixel 230 that outputs the imaging signal via the vertical transfer line 239 of the odd number column and outputs the sampled imaging signal to the output unit 31. The first sample-and-hold unit 242 includes a first sampling switch 251, a first sampling unit 252 (capacitor), a first output switch 253, a second sampling switch 254, a second sampling unit 255, and a second output switch 256.

The first sampling switch 251 has one end connected to the vertical transfer line 239 of odd number column, the other end connected to one end of the first output switch 253, and a gate connected to a signal line into which a drive pulse φNS is input from the timing generator 25.

The first sampling unit 252 has one end connected between the first sampling switch 251 and the first output switch 253, and the other end connected to the ground GND. In the case where the row selection pulse φX<M> and the drive pulse φR<M> are applied to the unit pixel 230, when the drive pulse φNS is applied to the gate of the first sampling switch 251, the first sampling unit 252 samples (holds) the noise signal from the unit pixel 230.

The first output switch 253 has one end connected to the first sampling switch 251, the other end connected to a first horizontal transfer line 259, and a gate into which a column selection pulse φH<M> is input from the horizontal scanning unit 244. When the column selection pulse φH<M> is applied to the gate of the first output switch 253, the first output switch 253 transfers the noise signal sampled by the first sampling unit 252 to the first horizontal transfer line 259.

The second sampling switch 254 has one end connected to the vertical transfer line 239, the other end connected to one end of the second output switch 256, and a gate connected to a signal line into which a drive pulse φSS is input from the timing generator 25.

The second sampling unit 255 has one end connected between the second sampling switch 254 and the second output switch 256, and the other end connected to the ground GND. In the case where the row selection pulse φX<M> and the drive pulse φT1<M> or drive pulse φT2<M> are applied to the unit pixel 230, when the drive pulse φSS is applied to the gate of the second sampling switch 254, the second sampling unit 255 samples (holds) the imaging signal from the unit pixel 230.

The second output switch 256 has one end connected to the second sampling switch 254, the other end connected to a second horizontal transfer line 260, and a gate into which the column selection pulse φH<M> is input from the horizontal scanning unit 244. When the column selection pulse φH<M> is applied to the gate of the second output switch 256, the second output switch 256 transfers the imaging signal sampled by the second sampling unit 255 to the second horizontal transfer line 260.

The second sample-and-hold unit 243 (sample-and-hold circuit) has the same configuration as the first sample-and-hold unit 242, samples the noise signal at the time of pixel reset in each unit pixel 230 which outputs the imaging signal via the vertical transfer line 239 of the even number column, and outputs the sampled noise signal to the output unit 31. Furthermore, the second sample-and-hold unit 243 samples the imaging signal photoelectrically converted by each unit pixel 230 that outputs the imaging signal via the vertical transfer line 239 of the even number column and outputs the sampled imaging signal to the output unit 31. The second sample-and-hold unit 243 includes a first sampling switch 251a, a first sampling unit 252a (capacitor), a first output switch 253a, a second sampling switch 254a, a second sampling unit 255a, and a second output switch 256a.

The first sampling switch 251a has one end connected to the vertical transfer line 239 of even number column, the other end connected to one end of the first output switch 253a, and a gate connected to a signal line into which a drive pulse φNS is input from the timing generator 25.

The first sampling unit 252a has one end connected between the first sampling switch 251a and the first output switch 253a, and the other end connected to the ground GND. In the case where the row selection pulse φX<M> and the drive pulse φR<M> are applied to the unit pixel 230, when the drive pulse φNS is applied to the gate of the first sampling switch 251a, the first sampling unit 252a samples (holds) the noise signal from the unit pixel 230.

The first output switch 253a has one end connected to the first sampling switch 251a, the other end connected to a third horizontal transfer line 261, and a gate into which the column selection pulse φH<M> is input from the horizontal scanning unit 244. When the column selection pulse φH<M> is applied to the gate of the first output switch 253a, the first output switch 253a transfers the noise signal sampled by the first sampling unit 252a to the third horizontal transfer line 261.

The second sampling switch 254a has one end connected to the vertical transfer line 239, the other end connected to one end of the second output switch 256a, and a gate connected to a signal line into which a drive pulse φSS is input from the timing generator 25.

The second sampling unit 255a has one end connected between the second sampling switch 254a and the second output switch 256a, and the other end connected to the ground GND. In the case where the row selection pulse φX<M> and the drive pulse φT1<M> or drive pulse φT2<M> are applied to the unit pixel 230, when the drive pulse φSS is applied to the gate of the second sampling switch 254a, the second sampling unit 255a samples (holds) the imaging signal from the unit pixel 230.

The second output switch 256a has one end connected to the second sampling switch 254a, the other end connected to a fourth horizontal transfer line 262, and a gate into which the column selection pulse φH<M> is input from the horizontal scanning unit 244. When the column selection pulse φH<M> is applied to the gate of the second output switch 256a, the second output switch 256a transfers the imaging signal sampled by the second sampling unit 255a to the fourth horizontal transfer line 262.

Based on the drive pulse (φH) supplied from the timing generator 25, the horizontal scanning unit 244 applies the column selection pulse φH<M> to the selected row of the light receiving unit 23 <M> (M = 1, 2, 3, ..., m), transfers the noise signal from each unit pixel 230 at the time of pixel reset in each unit pixel 230 to the first horizontal transfer line 259 via the first sample-and-hold unit 242 and output the noise signal, transfers the noise signal to the third horizontal transfer line 261 via the second sample-and-hold unit 243 and outputs the noise signal. Furthermore, based on the drive pulse φH<M> supplied from the timing generator 25, the horizontal scanning unit 244 applies the column selection pulse φH<M> to the selected row <M> of the light receiving unit 23, transfers the imaging signal photoelectrically converted by each unit pixel 230 to the second horizontal transfer line 260 via the first sample-and-hold unit 242 and output the noise signal, transfers the noise signal to the fourth horizontal transfer line 262 via the second sample-and-hold unit 243 and outputs the noise signal. In the first embodiment, the vertical scanning unit 241 and the horizontal scanning unit 244 function as the reading unit 24.

On the basis of the drive pulse φSW supplied from the timing generator 25, the switching unit 245 connects the first horizontal transfer line 259, the second horizontal transfer line 260, the third horizontal transfer line 261, and the fourth horizontal transfer line 262 to any one of the first output amplifier unit 311 and the second output amplifier unit 312 of the output unit 31 to be described later. The switching unit 245 includes a first selection switch 245a, a second selection switch 245b, a third selection switch 245c, a fourth selection switch 245d, a fifth selection switch 245e, a sixth selection switch 245f, a seventh selection switch 245g, an eighth selection switch 245h, and an inversion element 270.

The first selection switch 245a has one end connected to the first horizontal transfer line 259, the other end side connected to the first output amplifier unit 311 of the output unit 31 to be described later, and a gate connected to a signal line into which a drive pulse φSW is applied from the timing generator 25. The first selection switch 245a connects the first horizontal transfer line 259 and the first output amplifier unit 311 when the drive pulse φSW is applied to the gate.

The second selection switch 245b has one end connected to the second horizontal transfer line 260, the other end side connected to the first output amplifier unit 311 of the output unit 31 to be described later, and a gate connected to a signal line into which a drive pulse φSW is applied from the timing generator 25. The second selection switch 245b connects the second horizontal transfer line 260 and the first output amplifier unit 311 when the drive pulse φSW is applied to the gate.

The third selection switch 245c has one end connected to the third horizontal transfer line 261, the other end side connected to the second output amplifier unit 312 of the output unit 31 to be described later, and a gate connected to a signal line into which a drive pulse φSW is applied from the timing generator 25. The third selection switch 245c connects the third horizontal transfer line 261 and the second output amplifier unit 312 when the drive pulse φSW is applied to the gate.

The fourth selection switch 245d has one end connected to the fourth horizontal transfer line 262, the other end side connected to the second output amplifier unit 312 of the output unit 31 to be described later, and a gate connected to a signal line into which a drive pulse φSW is applied from the timing generator 25. The fourth selection switch 245d connects the fourth horizontal transfer line 262 and the second output amplifier unit 312 when the drive pulse φSW is applied to the gate.

The fifth selection switch 245e has one end connected to the first horizontal transfer line 259, the other end side connected to the second output amplifier unit 312 of the output unit 31 to be described later, and a gate connected to a signal line into which a drive pulse φSW is applied from the timing generator 25. The fifth selection switch 245e connects the first horizontal transfer line 259 and the second output amplifier unit 312 when the drive pulse φSW is applied to the gate.

The sixth selection switch 245f has one end connected to the second horizontal transfer line 260, the other end side connected to the second output amplifier unit 312 of the output unit 31 to be described later, and a gate connected to a signal line into which a drive pulse φSW is applied from the timing generator 25. The sixth selection switch 245f connects the second horizontal transfer line 260 and the second output amplifier unit 312 when the drive pulse φSW is applied to the gate.

The seventh selection switch 245g has one end connected to the third horizontal transfer line 261, the other end side connected to the first output amplifier unit 311 of the output unit 31 to be described later, and a gate connected to a signal line into which a drive pulse φSW is applied from the timing generator 25. The seventh selection switch 245g connects the third horizontal transfer line 261 and the first output amplifier unit 311 when the drive pulse φSW is applied to the gate.

The eighth selection switch 245h has one end connected to the fourth horizontal transfer line 262, the other end side connected to the first output amplifier unit 311 of the output unit 31 to be described later, and a gate connected to a signal line into which a drive pulse φSW is applied from the timing generator 25. The eighth selection switch 245h connects the fourth horizontal transfer line 262 and the first output amplifier unit 311 when the drive pulse φSW is applied to the gate.

The inversion element 270 inverts the pulse state of the drive pulse φSW supplied from the timing generator 25, and outputs the same to the fifth selection switch 245e, the sixth selection switch 245f, a seventh selection switch 235g, and the eighth selection switch 245h. For example, when the drive pulse φSW supplied from the timing generator 25 is in the ON state, the inversion element 270 outputs the drive pulse φSW inverted to the OFF state to the fifth selection switch 245e, the sixth selection switch 245f, the seventh selection switch 235g, and the eighth selection switch 245h. When the drive pulse φSW supplied from the timing generator 25 is in the OFF state, the inversion element 270 outputs the drive pulse φSW inverted to the ON state to the fifth selection switch 245e, the sixth selection switch 245f, the seventh selection switch 235g, and the eighth selection switch 245h.

On the basis of the drive pulse φhclr input from the timing generator 25, the horizontal reset unit 246 resets each of the first horizontal transfer line 259, the second horizontal transfer line 260, the third horizontal transfer line 261, and the fourth horizontal transfer line 262. The horizontal reset unit 246 includes a first horizontal reset transistor 271, a second horizontal reset transistor 272, a third horizontal reset transistor 273, and a fourth horizontal reset transistor 274.

The first horizontal reset transistor 271 has one end connected to a reference voltage VREF, the other end connected to the first horizontal transfer line 259, and a gate connected to a signal line into which the drive pulse φhclr is input from the timing generator 25. When the drive pulse φhclr is input to the gate of the first horizontal reset transistor 271 from the timing generator 25, the first horizontal reset transistor 271 is turned ON to reset the first horizontal transfer line 259.

The second horizontal reset transistor 272 has one end connected to a reference voltage VREF, the other end connected to the second horizontal transfer line 260, and a gate connected to a signal line into which the drive pulse φhclr is input from the timing generator 25. When the drive pulse φhclr is input to the gate of the second horizontal reset transistor 272 from the timing generator 25, the second horizontal reset transistor 272 is turned ON to reset the second horizontal transfer line 260.

The third horizontal reset transistor 273 has one end connected to a reference voltage VREF, the other end connected to the third horizontal transfer line 261, and a gate connected to a signal line into which the drive pulse φhclr is input from the timing generator 25. When the drive pulse φhclr is input to the gate of the third horizontal reset transistor 273 from the timing generator 25, the third horizontal reset transistor 273 is turned ON to reset the third horizontal transfer line 261.

The fourth horizontal reset transistor 274 has one end connected to a reference voltage VREF, the other end connected to the fourth horizontal transfer line 262, and a gate connected to a signal line into which the drive pulse φhclr is input from the timing generator 25. When the drive pulse φhclr is input to the gate of the fourth horizontal reset transistor 274 from the timing generator 25, the fourth horizontal reset transistor 274 is turned ON to reset the fourth horizontal transfer line 262.

The output unit 31 takes the difference between the noise signal and the imaging signal transferred from each of the first horizontal transfer line 259 to the fourth horizontal transfer line 262 via the switching unit 245, thereby outputting the imaging signal in which a noise has been removed, to the outside. The output unit 31 includes a first output amplifier unit 311 and a second output amplifier unit 312.

The first output amplifier unit 311 is configured using a differential amplifier. When the drive pulse φSW is in the ON state (High), by taking a difference between the imaging signal transferred from the first horizontal transfer line 259 via the first selection switch 245a and the noise signal transferred from the second horizontal transfer line 260 via the second selection switch 245b, and on the other hand, when the drive pulse φSW is in the OFF state (Low), by taking a difference between the imaging signal transferred from the third horizontal transfer line 261 via the seventh selection switch 245g and the noise signal transferred from the fourth horizontal transfer line 260 via the eighth selection switch 245h, the first output amplifier unit 311 outputs the imaging signal of odd number column from which noise has been removed, to the outside (Vout1).

The second output amplifier unit 312 is configured using a differential amplifier. When the drive pulse φSW is in the ON state (High), by taking a difference between the imaging signal transferred from the third horizontal transfer line 261 and the noise signal transferred from the fourth horizontal transfer line 262, and on the other hand, when the drive pulse φSW is in the OFF state (Low), by taking a difference between the imaging signal transferred from the first horizontal transfer line 259 and the noise signal transferred from the second horizontal transfer line 260, the second output amplifier unit 312 outputs the imaging signal of even number column from which noise has been removed, to the outside (Vout2).

In the light receiving unit 23 of the first chip 21, a large number of unit pixels 230 are arranged in a two-dimensional matrix. Each unit pixel 230 includes a photoelectric conversion element 231 (photodiode) and a photoelectric conversion element 232, a charge converter 233, a transfer transistor 234 (first transfer unit) and a transfer transistor 235, a charge conversion reset unit 236), a pixel source follower transistor 237, and a pixel output switch 238 (signal output unit).

In the description, one or more photoelectric conversion elements and a transfer transistor for transferring a signal charge from each photoelectric conversion element to the charge converter 233 are referred to as a unit cell. That is, the unit cell includes a set of one or more photoelectric conversion elements and transfer transistors, and each unit pixel 230 includes one unit cell. In the first embodiment, the unit pixel 230 includes two pixels (the photoelectric conversion element 231 and the photoelectric conversion element 232) shared by one vertical transfer line 239 (first transfer line). Alternatively, four or eight pixels may be shared by one vertical transfer line 239, for example. In the first embodiment, a plurality of unit pixels 230 for outputting imaging signals via the vertical transfer lines 239 of odd number column is referred to as pixel units G1, and a plurality of unit pixels 230 for outputting imaging signals via the vertical transfer lines 239 of the even number column is referred to as pixel units G2.

The photoelectric conversion element 231 and the photoelectric conversion element 232 photoelectrically convert incident light to a signal charge amount corresponding to the amount of light and accumulate them. The cathode side of the photoelectric conversion element 231 and the photoelectric conversion element 232 are connected to one end sides of the transfer transistor 234 and the transfer transistor 235, respectively, and the anode side is connected to the ground GND. The charge converter 233 is made up of a floating diffusion capacitance (FD), and converts the electric charge accumulated in the photoelectric conversion element 231 and the photoelectric conversion element 232 into a voltage.

The transfer transistor 234 and the transfer transistor 235 transfer charges from the photoelectric conversion element 231 and the photoelectric conversion element 232 to the charge converter 233. A signal line to which drive pulses (row selection pulses) φT1<M> and φT2<M> are supplied is connected to the gates of the transfer transistor 234 and the transfer transistor 235, and the charge converter 233 is connected to the other end. The transfer transistor 234 and the transfer transistor 235 are turned on when drive pulses φT1 and φT2 are supplied from the vertical scanning unit 241 via the signal line, and transfer the signal charge from the photoelectric conversion element 231 and the photoelectric conversion element 232 to the charge converter 233.

The charge conversion reset unit 236 resets the charge converter 233 to a predetermined potential. The charge conversion reset unit 236 has one end connected to the power supply voltage VDD, the other end connected to the charge converter 233, and a gate connected to a signal line to which the drive pulse φR<M> is supplied. When the drive pulse φR<M> is supplied from the vertical scanning unit 241 via the signal line, the charge conversion reset unit 236 is turned on to release the signal charge accumulated in the charge converter 233, and reset the charge converter 233 to a predetermined potential.

The pixel source follower transistor 237 has one end connected to the power supply voltage VDD, the other end connected to one end of the pixel output switch 238, and a gate to which a signal voltage-converted by the charge converter 233 (imaging signal or noise signal at a reset time) is input.

The pixel output switch 238 outputs the signal after voltage conversion by the charge converter 233 to the vertical transfer line 239. The pixel output switch 238 has the other end connected to the vertical transfer line 239, and a gate connected to a signal line to which the row selection pulse φX<M> is supplied. When the row selection pulse φX <M> is supplied from the vertical scanning unit 241 to the gate of the pixel output switch 238 via the signal line, the pixel output switch 238 is turned on, and the image signal or a signal at the reset time (noise signal) to the vertical transfer line 239.

### [Operation of Imaging Unit]

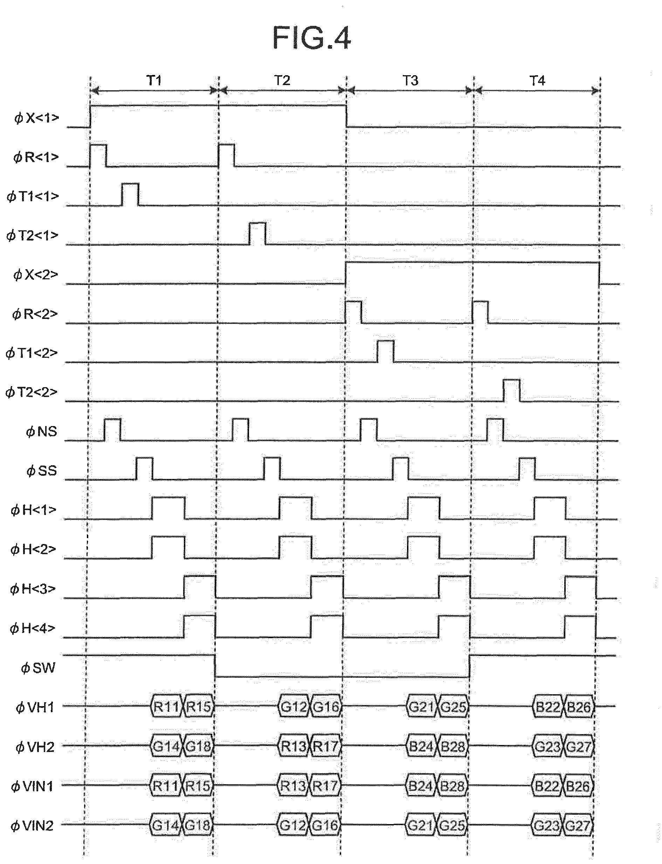

Next, the drive timing of the imaging unit 20 will be described. FIG. 4 is a timing chart illustrating the drive timing of the imaging unit 20. FIG. 4 illustrates timings of, in order from the top, a row selection pulse φX<1>, a drive pulse φR<1>, a drive pulse φT1<1>, a drive pulses φT2<1>, a row selection pulse φX<2>, a drive pulses φR<2>, a drive pulse φT1<2>, a drive pulse φT2<2>, a drive pulse φNS, a drive pulse φSS, column selection pulses φH<1> to <4>, a drive pulse φSW, a φVH1 indicating the type of the signal transferred to the first horizontal transfer line 259, a φVH2 indicating the type of the signal transferred to the third horizontal transfer line 261, a φVIN1 indicating the type of the signal input to the first output amplifier unit 311, and a φVIN2 indicating the type of the signal input to the second output amplifier unit 312.

### [Operation in Period T1]

As illustrated in FIG. 4, first, the timing generator 25 sets the row selection pulse φX<1> and the drive pulse φR<1> to the ON state (High). As a result, the charge conversion reset unit 236 in the first row is turned on to release the signal charge accumulated in the charge converter 233 in the first row, and reset the charge converter 233 in the first row to a predetermined potential.

Subsequently, the timing generator 25 sets the drive pulse φR <1> to the OFF state (Low), sets the drive pulse φNS to the ON state (High), causes the first sample-and-hold unit 242 to sample the noise signal input from the charge converter 233 in the first row via the vertical transfer line 239 of the odd number column, and causes the second sample-and-hold unit 243 to sample the noise signal input from the charge converter 233 in the first row via the vertical transfer line 239 of the even number column.

Thereafter, the timing generator 25 sets the drive pulse φNS to the OFF state (Low). As a result, the first sample-and-hold unit 242 completes the sampling of the noise signal in the odd number column of the first row. Further, the second sample-and-hold unit 243 completes the sampling of the noise signal in the even number column of the first row.

Subsequently, the timing generator 25 sets the drive pulse φT1<1> to the ON state (High) and sets the drive pulse φSS to the ON state (High). In this case, the transfer transistors 234 in each of the odd number column and the even number column in the first row are turned ON by receiving the drive pulse φT1<1> from the timing generator 25 at the gate thereof, transfers the signal charge from the photoelectric conversion element 231 (pixels R11, R15) of the odd number column in the first row to the charge converter 233, and transfers the signal charge from the photoelectric conversion elements 232 (pixels G14 and G18) in the even number column in the first row to the charge converter 233. At this time, the pixel output switch 238 of the odd number column causes the pixel source follower transistor 237 to output the imaging signal whose voltage is converted by the charge converter 233 to the vertical transfer line 239. The pixel output switch 238 of the even number column causes the pixel source follower transistor 237 to output the imaging signal after voltage conversion by the charge converter 233 to the vertical transfer line 239. Further, the first sample-and-hold unit 242 samples the imaging signal corresponding to the photoelectric conversion element 231 (pixels R11, R15) in the odd number column of the first row output from the vertical transfer line 239. In addition, the second sample-and-hold unit 243 samples the imaging signal corresponding to the photoelectric conversion element 232 (pixels G14, G18) in the even number column of the first row output from the vertical transfer line 239.

After that, the timing generator 25 sets the drive pulse φSS to the OFF state (Low), then sets the column selection pulse H<1> to the ON state, and sets the drive pulse φSW to the ON state (High). In this case, since the switching unit 245 is in the ON state, each of the imaging signal corresponding to the pixel R11 sampled by the first sample-and-hold unit 242 and the imaging signal corresponding to the pixel R15 is output to the first output amplifier unit 311 via the second horizontal transfer line 260 and the switching unit 245. Further, each of the imaging signal corresponding to the pixel G14 sampled by the second sample-and-hold unit 243 and the imaging signal corresponding to the pixel G18 is output to the second output amplifier unit 312 via the fourth horizontal transfer line 262 and the switching unit 245.

### [Operation in Period T2]

Subsequently, the timing generator 25 controls on/off operation of the drive pulse φR<1>, the drive pulse φT2<1>, the drive pulse φNS, and the drive pulse φSS. Accordingly, the first sample-and-hold unit 242 samples the imaging signal corresponding to the photoelectric conversion element 232 (pixels G12 and G16) in the even number column of the first row output from the vertical transfer line 239. Further, the second sample-and-hold unit 243 samples the imaging signal corresponding to the photoelectric conversion element 231 (pixels R13 and R17) in the odd number column of the first row output from the vertical transfer line 239.

Thereafter, the timing generator 25 sets the column selection pulse H<1> to the On state and sets the drive pulse φSW to the OFF state (Low). In this case, since the switching unit 245 is in the OFF state, each of the imaging signal corresponding to the pixel G12 sampled by the first sample-and-hold unit 242 and the imaging signal corresponding to the pixel G16 is output to the second output amplifier unit 312 via the second horizontal transfer line 260 and the switching unit 245. Further, each of the imaging signal corresponding to the pixel R13 sampled by the second sample-and-hold unit 243 and the imaging signal corresponding to the pixel R17 is output to the first output amplifier unit 311 via the fourth horizontal transfer line 262 and the switching unit 245.

### [Operation of Periods T3 and T4]

Subsequently, the timing generator 25 sets the row selection pulse φX<1> to the OFF state and sets the row selection pulse X<2> to the ON state (High). Thereafter, by controlling on/off operation of the drive pulse φR<2>, the drive pulse φT1<2>, the drive pulse φT2<2>, the drive pulse φNS, the drive pulse φSS, the column selection pulse φH<M> and the drive pulse φSW, the imaging signal corresponding to the B pixel in the second row is output from the first output amplifier unit 311 and the imaging signal corresponding to the G pixel is output only from the second output amplifier unit 312.

As described above, by controlling the on/off operation of the row selection pulse φX<M>, drive pulse φR<M>, drive pulse φT1<M>, drive pulse φT2<M>, drive pulse φNS, drive pulse φSS, column selection pulse φH<M>, and repeating the above operation, in the case of outputting the imaging signal from the pixel unit G1 and the pixel unit G2 to the first sample-and-hold unit 242 and the second sample-and-hold unit 243, the timing generator 25 controls the vertical scanning unit 241 so as to output the imaging signals in twice during one horizontal scanning period and output the imaging signals from the photoelectric conversion element 231 and the photoelectric conversion element 232 in which different filters are arranged on the light receiving surfaces. As a result, the imaging signal corresponding to the R pixel and the imaging signal corresponding to the B pixel are output from the first output amplifier unit 311, and the imaging signal corresponding to the G pixel is output only from the second output amplifier unit 312.

According to the first embodiment of the present invention described above, based on the type of filter in which the timing generator 25 is disposed on the light receiving surface of the photoelectric conversion element 231 and the photoelectric conversion element 232, since the transfer destination of the imaging signal transferred to the switching unit 245 from the first horizontal transfer line 259 to the fourth horizontal transfer line 262 is switched to either the first output amplifier unit 311 or the second output amplifier unit 312, it is possible to prevent variations from occurring in the image signal from the pixel in which the filter of the same color is arranged on the light receiving surface.

Furthermore, according to the first embodiment of the present invention, the timing generator 25 causes the switching unit 245 to switch the transfer destination of the imaging signal transferred from each of the first horizontal transfer line 259 to the fourth horizontal transfer line 262 so that the imaging signal (imaging signal corresponding to G pixels) output from the photoelectric conversion element 232 in which filters of the same type are arranged on the light receiving surface is transferred to the same second output amplifier unit 312, it is possible to further improve the frame rate while preventing variations between the channels.

In the first embodiment of the present invention, the noise signal and the imaging signal at the reset time are sampled by each of the first sample-and-hold unit 242 and the second sample-and-hold unit 243. However, for example, a CDS circuit may be provided between the unit pixel 230 and the first sample-and-hold unit 242 or the second sample-and-hold unit 243, and the difference between the noise signal and the imaging signal may be sampled.

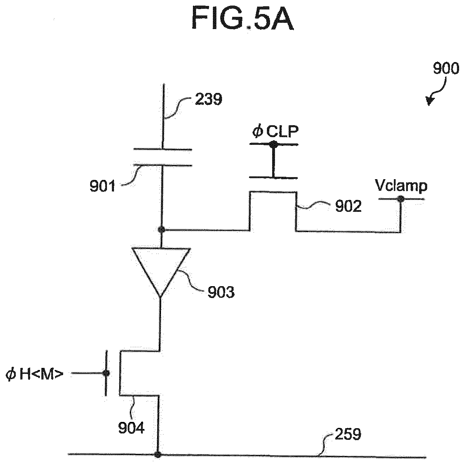

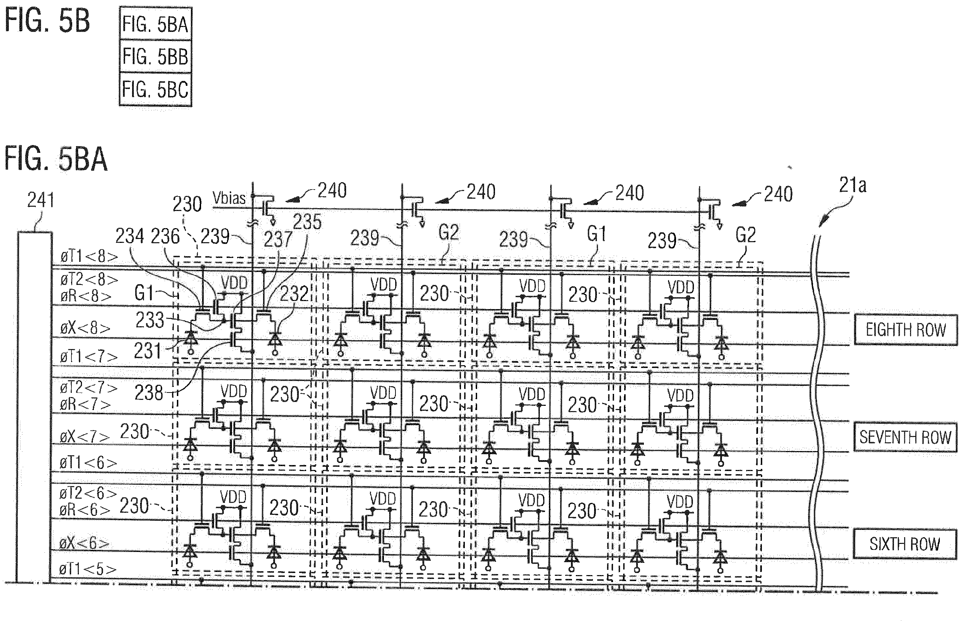

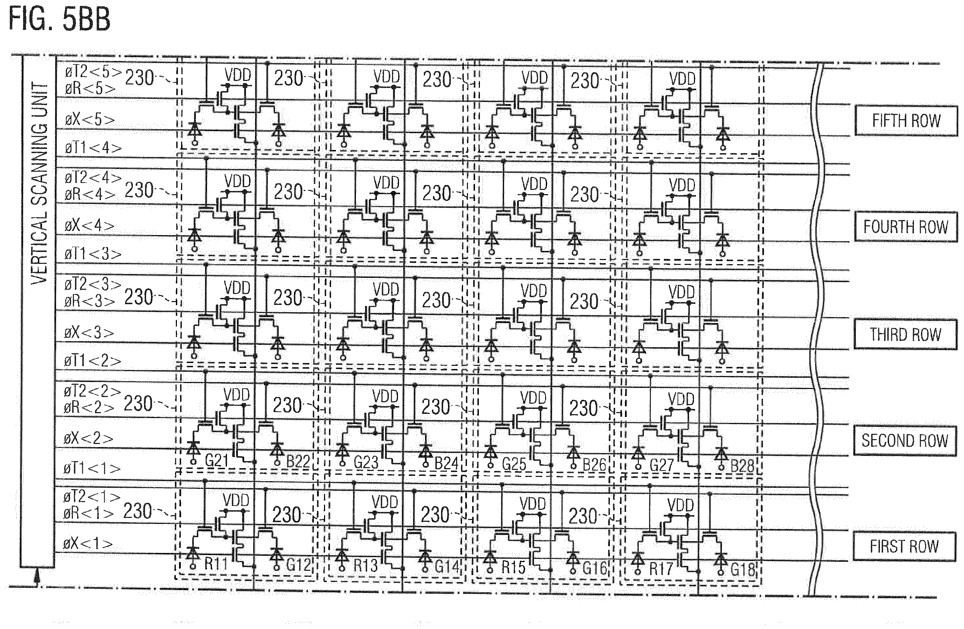

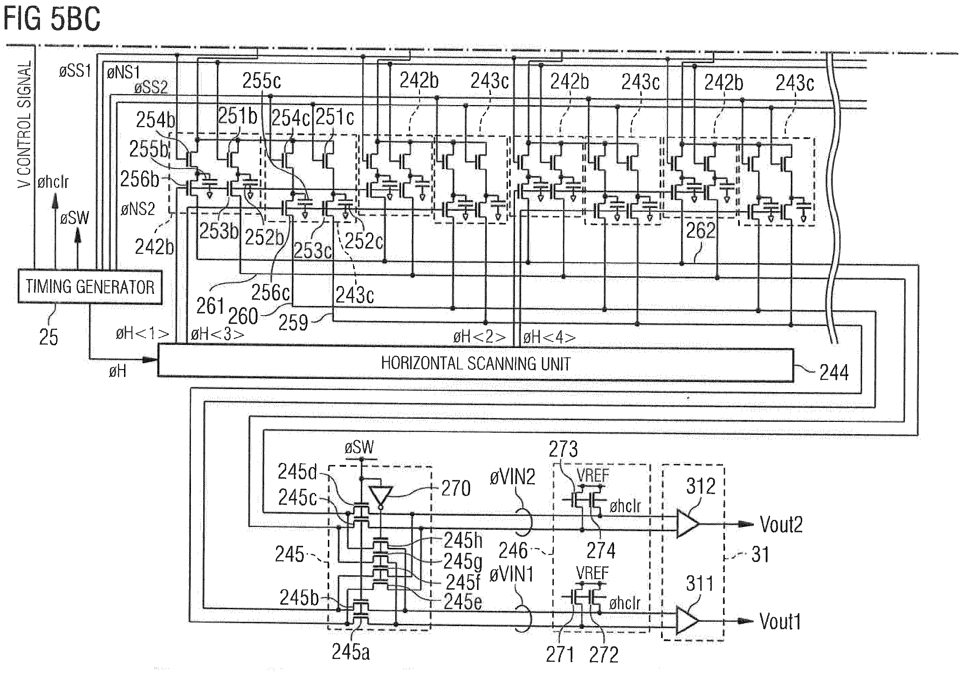

Furthermore, in the first embodiment of the present invention, a CDS circuit having a sample and hold function may be used. FIG. 5A is a circuit diagram of a CDS circuit according to a modification of the first embodiment of the present invention. A CDS circuit 900 illustrated in FIG. 5A is configured by combining a clamp circuit constituted by a clamp capacitor 901, a clamp switch 902, and a buffer amplifier 903 (for example, a source follower circuit). The CDS circuit 900 illustrated in FIG. 5A holds the electric charge corresponding to the noise signal output to the vertical transfer line 239 in the clamp capacitor 901, and then outputs the imaging signal to the vertical transfer line 239, thereby outputting the difference between the noise signal and the imaging signal. At this time, by controlling the row selection pulse φX<M> and the drive pulse φT1<M> or the drive pulse φT2<M>, during the period in which the imaging signal is output from the photoelectric conversion element 231 and the photoelectric conversion element 232 via an output switch 904, the timing generator 25 can continuously output the difference between the noise signal and the imaging signal without separately providing a sample-and-hold circuit.

In the first embodiment of the present invention, the switching unit 245 is provided between each of the first horizontal transfer line 259 to the fourth horizontal transfer line 262 and the output unit 31. However, for example, the switching unit 245 is provided for each pixel unit G1 column and for each pixel unit G2 column, so that for example, the imaging signal from the first sample-and-hold unit 242 may be transferred to any one of the first horizontal transfer line 259 to the fourth horizontal transfer line 262. Needless to say, the switching unit 245 may be provided between the pixel unit G1 and the first sample-and-hold unit 242 and between the pixel unit G2 and the second sample-and-hold unit 243.

### (Second Embodiment)

Next, the second embodiment of the present invention will be described. The endoscope system according to the second embodiment is different from the configuration of the first chip 21 in the endoscope system 1 according to the above-described first embodiment. Specifically, in the first chip 21 according to the above-described first embodiment, one sample-and-hold unit (sample-and-hold circuit) is provided for each pixel unit group. However, in a first chip 21 according to the second embodiment , two sample-and-hold units (sample and hold circuits) are provided for each pixel unit. Hereinafter, the operation of the imaging unit according to the second embodiment will be described after describing the configuration of the first chip according to the second embodiment. The same elements as those of the endoscope system 1 according to the above first embodiment are denoted by the same reference signs, and the explanation thereof is omitted.

### [Configuration of First Chip]

FIG. 5B is a circuit diagram illustrating a configuration of a first chip according to a second embodiment of the present invention. The first chip 21a illustrated in FIG. 5B includes a first sample-and-hold unit 242b and a second sample-and-hold unit 243c in place of each of the first sample-and-hold unit 242 and the second sample-and-hold unit 243 of the first chip 21 according to the above-described first embodiment.

When the drive pulse φR<M> is applied, the first sample-and-hold unit 242b samples the noise signal at the pixel reset time in the unit pixel 230, and outputs the sampled noise signal to the output unit 31. Furthermore, when the drive pulse φT1<M> is applied, the first sample-and-hold unit 242b samples the imaging signal photoelectrically converted by each unit pixel 230, and outputs the sampled imaging signal to the output unit 31. The first sample-and-hold unit 242b includes a first sampling switch 251b, a first sampling unit 252b (capacitor), a first output switch 253b, a second sampling switch 254b, a second sampling unit 255b, and a second output switch 256b.

The first sampling switch 251b has one end connected to the vertical transfer line 239, the other end connected to one end of the first output switch 253b, and a gate connected to a signal line to which the drive pulse φNS1 is input from the timing generator 25.

The first sampling unit 252b has one end connected between the first sampling switch 251b and the first output switch 253b and the other end side connected to the ground GND. In the case where the row selection pulse φX<M> and the drive pulse φR<M> are applied to the unit pixel 230, when the drive pulse φNS1 is applied to the gate of the first sampling switch 251b, the first sampling unit 252b samples (holds) the noise signal from the unit pixel 230.

The first output switch 253b has one end connected to the first sampling switch 251b, the other end connected to the third horizontal transfer line 261, and a gate into which the column selection pulse φH<M> is input from the horizontal scanning unit 244. When the column selection pulse φH<M> is applied to the gate of the first output switch 253a, the first output switch 253b transfers the noise signal sampled by the first sampling unit 252b to the third horizontal transfer line 261.

The second sampling switch 254b has one end connected to the vertical transfer line 239, the other end connected to one end of the second output switch 256b, and a gate connected to a signal line to which the drive pulse φSS1 is input from the timing generator 25.

The second sampling unit 255b has one end connected between the second sampling switch 254b and the second output switch 256b and the other end side connected to the ground GND. In the case where the row selection pulse φX<M> and the drive pulse φT1<M> or drive pulse φT1<M> are applied to the unit pixel 230, when the drive pulse φSS1 is applied to the gate of the second sampling switch 254b, the second sampling unit 255b samples (holds) the imaging signal from the unit pixel 230.

The second output switch 256b has one end connected to the second sampling switch 254b, the other end connected to the fourth horizontal transfer line 262, and a gate into which the column selection pulse φH<M> is input from the horizontal scanning unit 244. When the column selection pulse φH<M> is applied to the gate of the second output switch 256b, the second output switch 256b transfers the imaging signal sampled by the second sampling unit 255b to the horizontal transfer line.

The second sample-and-hold unit 243c (sample-and-hold circuit) has the same configuration as the first sample-and-hold unit 242b, and when the drive pulse φR<M> is applied, samples the noise signal at the pixel reset time in each unit pixel 230, and outputs the sampled noise signal to the output unit 31. Furthermore, when the drive pulse φT2<M> is applied, the second sample-and-hold unit 243c samples the imaging signal photoelectrically converted by each unit pixel 230, and outputs the sampled imaging signal to the output unit 31. The second sample-and-hold unit 243c includes a first sampling switch 251c, a first sampling unit 252c (capacitor), a first output switch 253c, a second sampling switch 254c, a second sampling unit 255c, and a second output switch 256c.

The first sampling switch 251c has one end connected to the vertical transfer line 239, the other end connected to one end of the first output switch 253c, and a gate connected to a signal line to which the drive pulse φNS2 is input from the timing generator 25.

The first sampling unit 252c has one end connected between the first sampling switch 251c and the first output switch 253c, and the other end connected to the ground GND. In the case where the row selection pulse φX<M> and the drive pulse φR<M> are applied to the unit pixel 230, when the drive pulse φNS2 is applied to the gate of the first sampling switch 251c, the first sampling unit 252c samples (holds) the noise signal from the unit pixel 230.

The first output switch 253c has one end connected to the first sampling switch 251c, the other end connected to the first horizontal transfer line 259, and a gate into which the column selection pulse φH<M> is input from the horizontal scanning unit 244. When the column selection pulse φH<M> is applied to the gate of the first output switch 253c, the first output switch 253c transfers the noise signal sampled by the first sampling unit 252c to the horizontal transfer line.

The second sampling switch 254c has one end connected to the vertical transfer line 239, the other end connected to one end of the second output switch 256c, and a gate connected to a signal line to which the drive pulse φSS2 is input from the timing generator 25.

The second sampling unit 255c has one end connected between the second sampling switch 254c and the second output switch 256c, and the other end connected to the ground GND. In the case where the row selection pulse φX<M> and the drive pulse φT1<M> or drive pulse φT1<M> are applied to the unit pixel 230, when the drive pulse φSS2 is applied to the gate of the second sampling switch 254c, the second sampling unit 255c samples (holds) the imaging signal from the unit pixel 230.

The second output switch 256c has one end connected to the second sampling switch 254c, the other end connected to the second horizontal transfer line 260, and a gate into which the column selection pulse φH<M> is input from the horizontal scanning unit 244. When the column selection pulse φH<M> is applied to the gate of the second output switch 256c, the first output switch 256c transfers the imaging signal sampled by the second sampling unit 255c to the horizontal transfer line.

### [Operation of Imaging Unit]

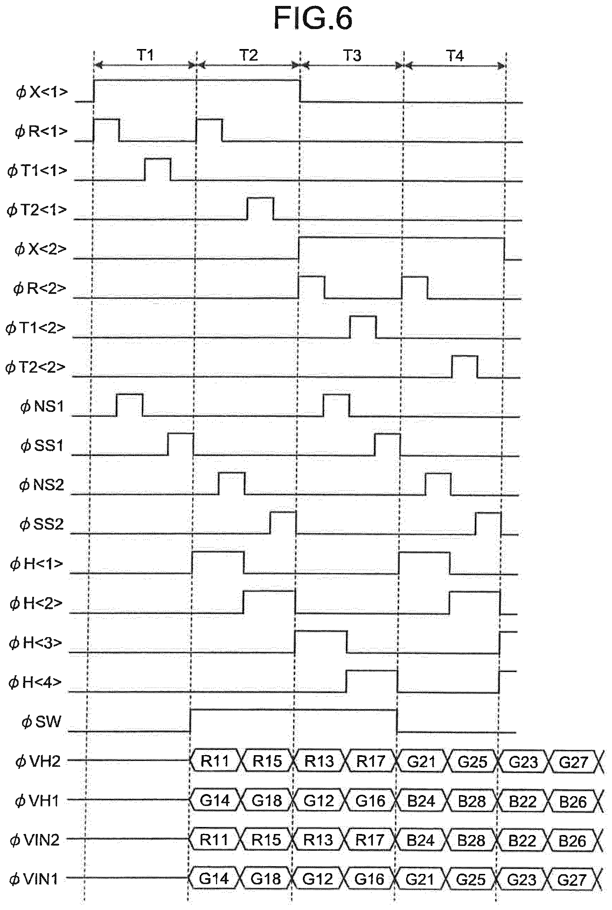

Next, the drive timing of the imaging unit 20 will be described. FIG. 6 is a timing chart illustrating the drive timing of the imaging unit 20. FIG. 6 illustrates timings of, in order from the top, a row selection pulse φX<1>, a drive pulse φR<1>, a drive pulse φT1<1>, a drive pulses φT2<1>, a row selection pulse φX<2>, a drive pulses φR<2>, a drive pulse φT1<2>, a drive pulse φT2<2>, a drive pulse φNS1, a drive pulse φSS1, a drive pulse φNS2, a drive pulse φSS2, column selection pulses φH<1> to <4>, a drive pulse φSW, a φVH1 indicating the type of the signal transferred to the first horizontal transfer line 259, a φVH2 indicating the type of the signal transferred to the third horizontal transfer line 261, a φVIN1 indicating the type of the signal input to the first output amplifier unit 311, and a φVIN2 indicating the type of the signal input to the second output amplifier unit 312.

### [Operation in Period T1]

As illustrated in FIG. 6, first, the timing generator 25 sets the row selection pulse φX<1> and the drive pulse φR<1> to the ON state (High). As a result, the charge conversion reset unit 236 in the first row is turned on to release the signal charge accumulated in the charge converter 233 in the first row, and reset the charge converter 233 in the first row to a predetermined potential.

Subsequently, the timing generator 25 sets the drive pulse φR<1> to the OFF state (Low), sets the drive pulse φNS1 to the ON state (High), and causes the first sample-and-hold unit 242b to sample the noise signal input from the charge converter 233 in the first row via the vertical transfer line 239.

Thereafter, the timing generator 25 sets the drive pulse φNS1 to the OFF state (Low). As a result, the sampling of the noise signal in the first row of the first sample-and-hold unit 242b is completed.

Subsequently, the timing generator 25 sets the drive pulse φT1<1> to the ON state (High). In this case, the transfer transistors 234 in each of the odd number column and the even number column in the first row are turned ON by receiving the drive pulse φT1<1> from the timing generator 25 at the gate thereof, transfers the signal charge from the photoelectric conversion element 231 (pixels R11, R15) of the odd number column in the first row to the charge converter 233, and transfers the signal charge from the photoelectric conversion elements 232 (pixels G14 and G18) in the even number column in the first row to the charge converter 233. At this time, the pixel output switch 238 of the odd number column causes the pixel source follower transistor 237 to output the imaging signal whose voltage is converted by the charge converter 233 to the vertical transfer line 239. The pixel output switch 238 of the even number column causes the pixel source follower transistor 237 to output the imaging signal after voltage conversion by the charge converter 233 to the vertical transfer line 239. Thereafter, by setting the drive pulse φSS1 to the ON state (High), the first sample-and-hold unit 242b samples the imaging signal corresponding to the photoelectric conversion element 231 and the photoelectric conversion element 232 (pixels R11, R15, G14, G18) of the first row output from the vertical transfer line 239.

### [Operation in Period T2]

After that, the timing generator 25 sets the drive pulse φSS1 to the OFF state (Low), then sets the column selection pulse H<1> to the ON state, and sets the drive pulse φ SW to the ON state (High). In this case, since the switching unit 245 is in the ON state, each of the imaging signals corresponding to the pixel R11 sampled by the first sample-and-hold unit 242b and the imaging signal corresponding to the pixel R15 is output to the second output amplifier unit 312 via the fourth horizontal transfer line 262 and the switching unit 245. Further, each of the imaging signals corresponding to the pixel G14 sampled by the first sample-and-hold unit 242b and the imaging signal corresponding to the pixel G18 is output to the first output amplifier unit 311 via the second horizontal transfer line 260 and the switching unit 245. At this time, the timing generator 25 controls on/off operations of the drive pulse φR<1>, the drive pulse φT2<1>, the drive pulse φNS2, and the drive pulse φSS2. As a result, the second sample-and-hold unit 243c samples the imaging signal corresponding to the photoelectric conversion element 231 (pixels R13, R17) in the odd number column of the first row output from the vertical transfer line 239. Furthermore, the second sample-and-hold unit 243c samples the imaging signal corresponding to the photoelectric conversion element 232 (pixels G12, G16) in the even number column of the first row output from the vertical transfer line 239.

### [Operation in Period T3]

Subsequently, after setting the row selection pulse φX<1> and the drive pulse φSS2 to the OFF state (Low), the timing generator 25 sets the column selection pulse H<3> to the ON state (High). In this case, since the switching unit 245 is in the ON state, each of the imaging signals corresponding to the pixel R13 sampled by the second sample-and-hold unit 243c and the imaging signal corresponding to the pixel R17 is output to the second output amplifier unit 312 via the fourth horizontal transfer line 262 and the switching unit 245. Furthermore, each of the imaging signals corresponding to the pixel G12 sampled by the second sample-and-hold unit 243c and the imaging signal corresponding to the pixel G16 is output to the first output amplifier unit 311 via the second horizontal transfer line 260 and the switching unit 245. At this time, the timing generator 25 controls on/off operations of the row selection pulse φX<2>, the drive pulse φR<2>, the drive pulse φT1<2>, the drive pulse φNS1, and the drive pulse φSS1. Accordingly, the first sample-and-hold unit 242b samples the imaging signal corresponding to the photoelectric conversion element 231 (pixels G21 and G25) in the odd number column of the second row output from the vertical transfer line 239. Furthermore, the first sample-and-hold unit 242b samples the imaging signal corresponding to the photoelectric conversion element 232 (pixels B24 and B28) in the even number column of the second row output from the vertical transfer line 239.

### [Operation in Period T4]

After that, the timing generator 25 sets the drive pulse φSS1 to the OFF state (Low), then sets the column selection pulse H<1> to the ON state (High), and sets the drive pulse φSW to the OFF state (Low). In this case, since the switching unit 245 is in the OFF state, each of the imaging signals corresponding to the pixel B24 sampled by the first sample-and-hold unit 242b and the imaging signal corresponding to the pixel B28 is output to the second output amplifier unit 312 via the second horizontal transfer line 260 and the switching unit 245. Further, each of the imaging signals corresponding to the pixel G21 sampled by the first sample-and-hold unit 242b and the imaging signal corresponding to the pixel G25 is output to the first output amplifier unit 311 via the fourth horizontal transfer line 262 and the switching unit 245. At this time, the timing generator 25 controls the on/off operation of the drive pulse φR<2>, the drive pulse φT2<2>, the drive pulse φNS2, and the drive pulse φSS2. Accordingly, the second sample-and-hold unit 243c samples the imaging signal corresponding to the photoelectric conversion element 231 (pixels G23 and G27) in the odd number column of the second row output from the vertical transfer line 239. Furthermore, the second sample-and-hold unit 243c samples the imaging signal corresponding to the photoelectric conversion element 232 (pixels B22 and B26) in the even number column of the second row output from the vertical transfer line 239.

As described above, by controlling the on/off operation of the row selection pulse φX<M>, drive pulse φR<M>, drive pulse φT1<M>, drive pulse φT2<M>, drive pulse φNS, drive pulse φSS, column selection pulse φH<M>, and drive pulse φSW, and repeating the above operation, in the case of outputting the imaging signal from the pixel unit G1 and the pixel unit G2 to the first sample-and-hold unit 242b and the second sample-and-hold unit 243c, the timing generator 25 controls the vertical scanning unit 241 so as to output the imaging signals in twice during one horizontal scanning period and output the imaging signals from the photoelectric conversion element 231 and the photoelectric conversion element 232 in which different filters are arranged on the light receiving surfaces. As a result, the imaging signal corresponding to the R pixel and the imaging signal corresponding to the B pixel are output from the second output amplifier unit 312. Furthermore, the imaging signal corresponding to the G pixel is output only from the first output amplifier unit 311.

According to the second embodiment of the present invention described above, based on the type of filter in which the timing generator 25 is disposed on the light receiving surface of the photoelectric conversion element 231 and the photoelectric conversion element 232, since the transfer destination of the imaging signal transferred to the switching unit 245 from the first horizontal transfer line 259 to the fourth horizontal transfer line 262 is switched to either the first output amplifier unit 311 or the second output amplifier unit 312, it is possible to prevent variations from occurring in the image signal from the pixel in which the filter of the same color is arranged on the light receiving surface.

Further, according to the second embodiment of the present invention, when the timing generator 25 causes the pixel unit G1 and the pixel unit G2 to output an imaging signal to the first sample-and-hold unit 242b and the second sample-and-hold unit 243c, by controlling the vertical scanning unit 241 so as to output the imaging signals in twice during one horizontal scanning period and output the imaging signals from the photoelectric conversion element 231 and the photoelectric conversion element 232 in which different filters are arranged on the light receiving surfaces, the timing generator 25 causes the second output amplifier unit 312 to output the imaging signal corresponding to the R pixel and the imaging signal corresponding to the B pixel, and the timing generator 25 causes the first output amplifier unit 311 to output only the imaging signal corresponding to the G pixel; therefore, since all the reading periods of one row can be assigned to horizontal scanning, it is possible to improve the frame rate without increasing the speed of the first output amplifier unit 311 and the second output amplifier unit 312.

### (Modification of Second Embodiment)

Next, a modification of the second embodiment of the present invention will be described. In the above-described second embodiment, the switching unit 245 is provided between each horizontal transfer line and the output unit 31; however, in the modification of the second embodiment, the switching unit is provided between each sample-and-hold unit and each horizontal transfer line. Hereinafter, the configuration of the first chip according to the modification of the second embodiment will be described. The same elements as those of the endoscope system 1 according to the above second embodiment are denoted by the same reference signs, and the explanation thereof is omitted.

### [Configuration of First Chip]

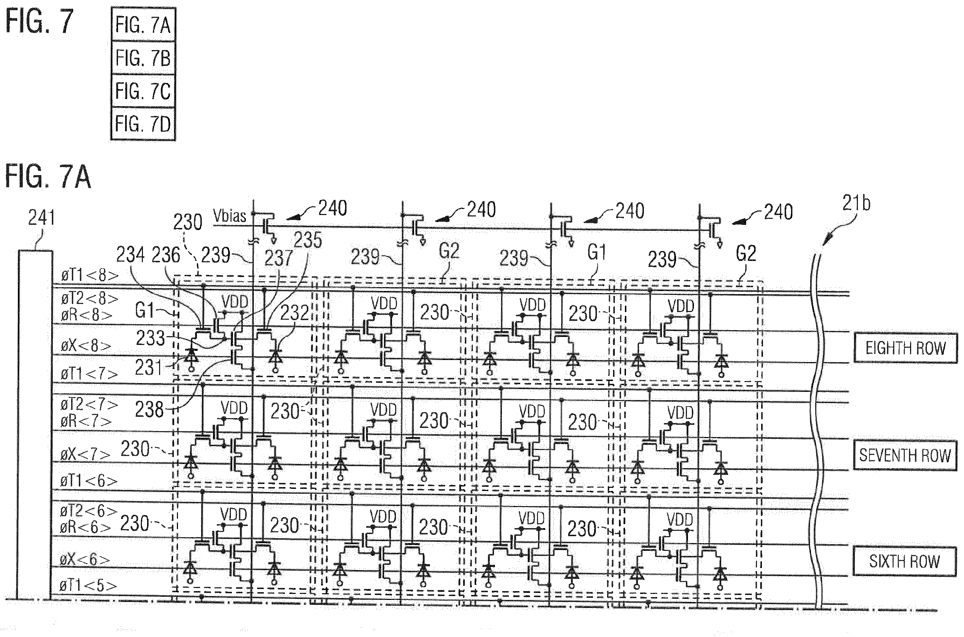

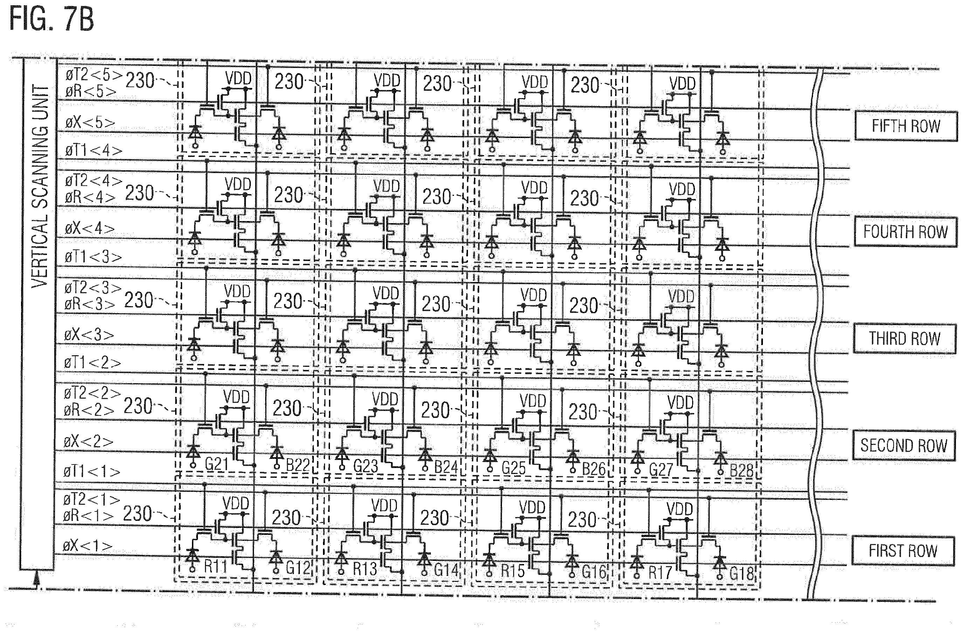

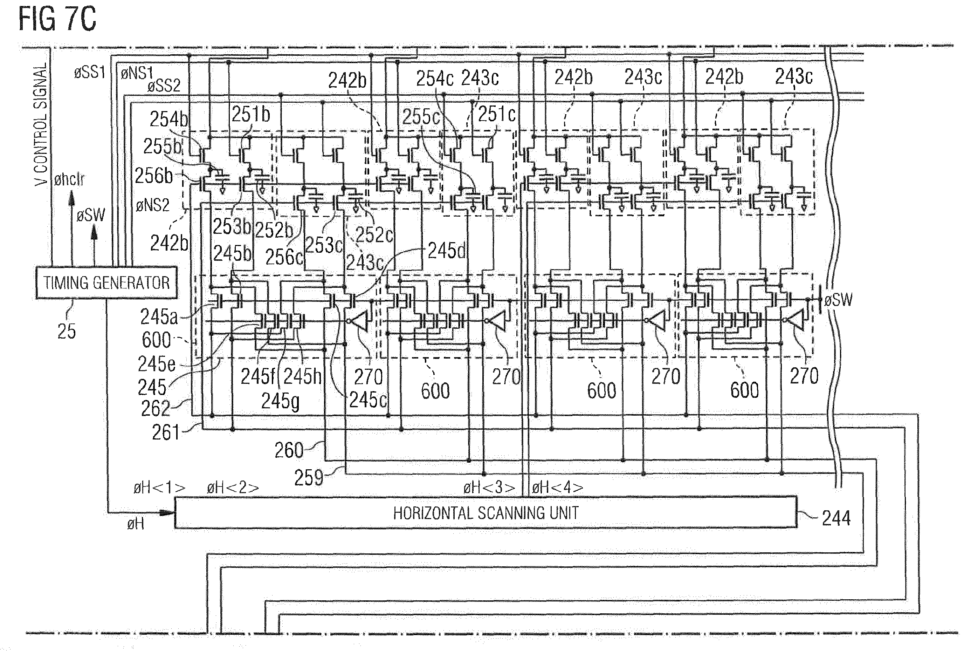

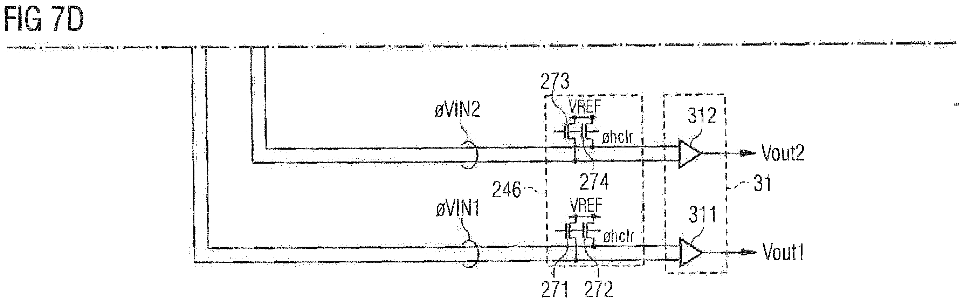

FIG. 7 is a circuit diagram illustrating a configuration of a first chip according to a modification of the second embodiment of the present invention. In place of the switching unit 245 relating to the first chip 21a according to the above-described second embodiment, the first chip 21b illustrated in FIG. 7 includes a plurality of switching units 600 provided in each pixel unit group and switching the output of the imaging signal output from the first sample-and-hold unit 242b and the second sample-and-hold unit 243c.

The switching unit 600 has the same configuration as the switching unit 245 according to the above-described second embodiment, and based on the drive pulse φSW input from the timing generator 25, outputs the imaging signal output from the first sample-and-hold unit 242b and the second sample-and-hold unit 243c to the second horizontal transfer line 260 or the fourth horizontal transfer line 262. For example, when the imaging signal corresponding to the R pixel and the imaging signal corresponding to the B pixel are output from the first sample-and-hold unit 242b, the switching unit 600 connects the first sample-and-hold unit 242b and the fourth horizontal transfer line 262, and outputs each of the imaging signal corresponding to the R pixel and the imaging signal corresponding to the B pixel to the second output amplifier unit 312. On the other hand, when the imaging signal corresponding to the G pixel is output from the first sample-and-hold unit 242b, the switching unit 600 connects the first sample-and-hold unit 242b and the second horizontal transfer line 260, and outputs the imaging signal corresponding to the G pixel to the first output amplifier unit 311.

According to the modification of the second embodiment of the present invention described above, the same effect as that of the above-described second embodiment is obtained.

In the modification of the second embodiment of the present invention, the switching unit 600 is provided between the sample-and-hold unit and the horizontal transfer line. However, for example, the switching unit 600 may be provided between the unit pixel 230 and the sample-and-hold unit.

### (Third Embodiment)

Next, the third embodiment of the present invention will be described. The endoscope system according to the third embodiment is different from the configuration of the first chip 21a in the endoscope system 1 according to the above-described second embodiment. Specifically, in the above-described first chip 21a according to the second embodiment, the output unit 31 amplifies the imaging signal and outputs the amplified imaging signal to the outside. However, the first chip according to the third embodiment performs a sampling operation in which the output unit holds the imaging signal, and an A/D conversion operation (processing operation) for A/D converting the sampled imaging signal. Hereinafter, the operation of the imaging unit according to the third embodiment will be described after describing the configuration of the first chip according to the third embodiment. The same elements as those of the endoscope system 1 according to the above second embodiment are denoted by the same reference sings, and the explanation thereof is omitted.

### [Configuration of First Chip]

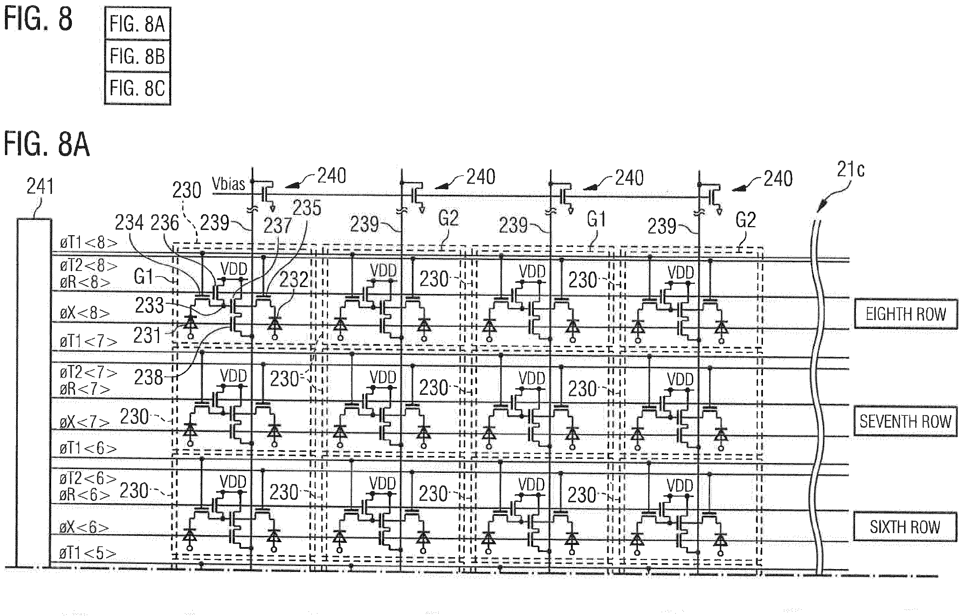

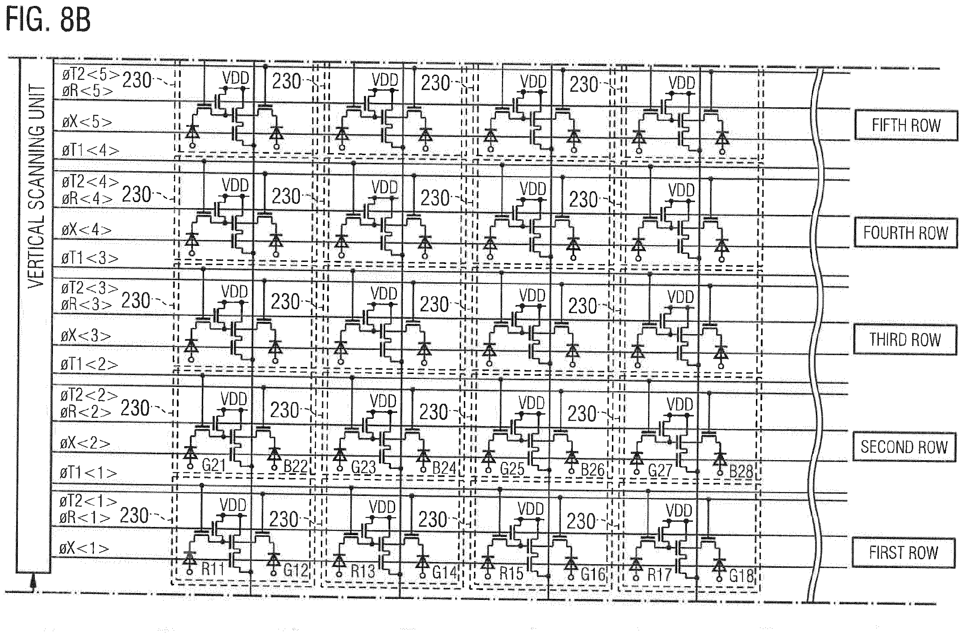

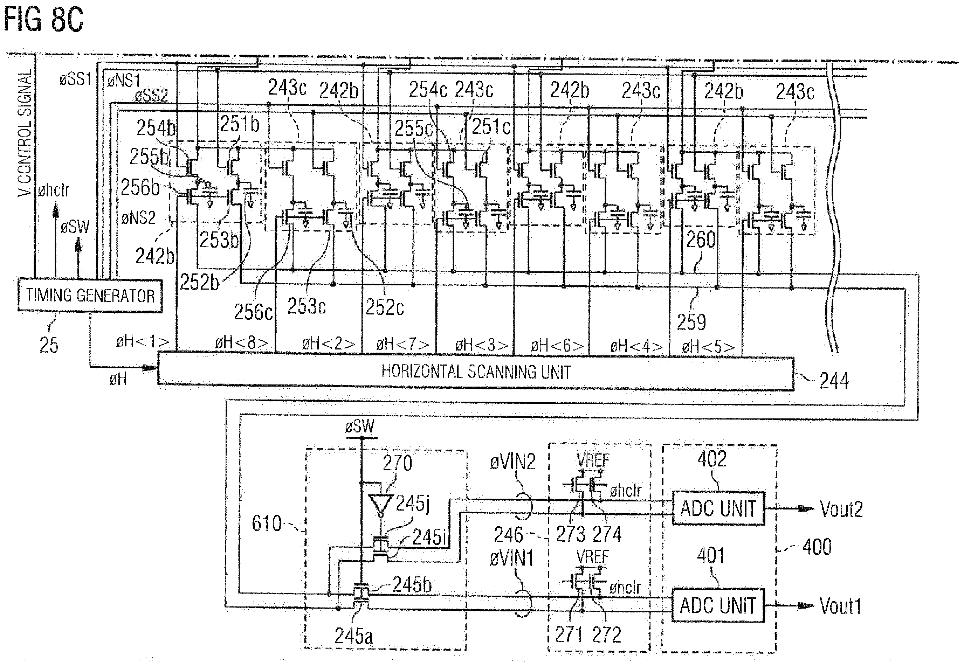

FIG. 8 is a circuit diagram illustrating the configuration of the first chip according to the third embodiment of the present invention. The first chip 21c illustrated in FIG. 8 includes a switching unit 610 and an output unit 400 in place of the switching unit 245 and the output unit 31 of the first chip 21a according to the above-described second embodiment. Furthermore, in the first chip 21 c, the third horizontal transfer line 261 and the fourth horizontal transfer line 262 are omitted from the configuration of the first chip 21a according to the above-described second embodiment, and the second sample-and-hold unit 243 c is connected to the first horizontal transfer line 259 and the second horizontal transfer line 260.

On the basis of the drive pulse φSW supplied from the timing generator 25, the switching unit 610 connects the first horizontal transfer line 259 and the second horizontal transfer line 260 to one of a first ADC unit 401 and a second ADC unit 402 of the output unit 400 to be described later. The switching unit 610 includes the first selection switch 245a, the second selection switch 245b, a third selection switch 245i, and a fourth selection switch 245j .

The third selection switch 245i has one end connected to the first horizontal transfer line 259, the other end side connected to the second ADC unit 402 of the output unit 400 to be described later, and a gate connected to a signal line into which a drive pulse φSW is applied from the timing generator 25.

The fourth selection switch 245j has one end connected to the second horizontal transfer line 260, the other end side connected to the second ADC unit 402 of the output unit 400 to be described later, and a gate connected to a signal line into which a drive pulse φSW is applied from the timing generator 25.

The output unit 400 samples the imaging signal input from the first horizontal transfer line 259 or the second horizontal transfer line 260 via the switching unit 600 and performs A/D conversion on the sampled imaging signal, generates a digital imaging signal, and outputs the digital imaging signal to the outside. The output unit 400 includes the first ADC unit 401 and the second ADC unit 402.

The first ADC unit 401 samples the imaging signal input from the first horizontal transfer line 259, performs A/D conversion on the sampled imaging signal, generates a digital imaging signal, and outputs the digital imaging signal to the outside (Vout1).

The second ADC unit 402 samples the imaging signal input from the second horizontal transfer line 260, performs A/D conversion on the sampled imaging signal, generates a digital imaging signal, and outputs the digital imaging signal to the outside (Vout2).

### [Operation of Imaging Unit]

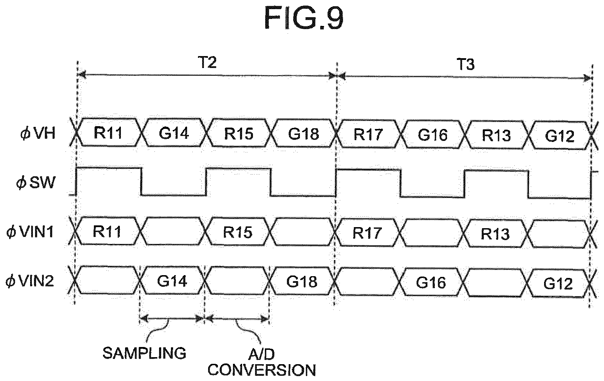

Next, the drive timing of the imaging unit 20 will be described. FIG. 9 is a timing chart illustrating the drive timing of the imaging unit 20. FIG. 9 illustrates timings of, in order from the top, φVH1 indicating the type of the signal transferred to the first horizontal transfer line 259, φVH2 indicating the type of the signal transferred to the second horizontal transfer line 260, φVIN1 indicating the type of the signal input to the first ADC unit 401, and φVIN2 indicating the type of the signal input to the second ADC unit 402. Note that the imaging unit 20 according to the third embodiment performs the same operation as the imaging unit 20 according to the above-described second embodiment, and only the operations of the switching unit 610 and the output unit 400 are different. Since the other operation is the same as that of the above-described second embodiment, description thereof will be omitted.

### [Operation of Periods T2 and T3]

As illustrated in FIG. 9, the timing generator 25 performs on/off operation of the column selection pulse φH<M> and controls the horizontal scanning unit 244. Accordingly, the imaging signal (R pixel) corresponding to the pixel R11 and the imaging signal (G pixel) corresponding to the pixel G14 are alternately read out by selecting the first sample-and-hold unit 242b or the second sample-and-hold unit 243c from which the imaging signal is read out. On the basis of the drive pulse φSW input from the timing generator 25, the switching unit 610 switches, the imaging signal input from the first horizontal transfer line 259 or the second horizontal transfer line 260 to the first ADC unit 401 or the second ADC unit 402 and output the imaging signal. At this time, the first ADC unit 401 or the second ADC unit 402 performs the sampling operation of the input signal in one of the input signal φVIN1 and the input signal φVIN2, performs the A/D conversion operation in the other period, and output the signal.

Further, according to the third embodiment of the present invention described above, when the timing generator 25 causes the pixel unit G1 and the pixel unit G2 to output an imaging signal to the first sample-and-hold unit 242b and the second sample-and-hold unit 243c, by controlling the vertical scanning unit 241 so as to output the imaging signals in twice during one horizontal scanning period and output the imaging signals from the photoelectric conversion element 231 and the photoelectric conversion element 232 in which different filters are arranged on the light receiving surfaces, the timing generator 25 causes the first ADC unit 401 to output the imaging signal corresponding to the R pixel and the imaging signal corresponding to the B pixel, and the timing generator 25 causes the second ADC unit 402 to output only the imaging signal corresponding to the G pixel; therefore, since all the reading periods of one row can be assigned to horizontal scanning, it is possible to improve the frame rate without increasing the speed of the first ADC unit 401 and the second ADC unit 402.

In the third embodiment of the present invention, each of the first amplifier for amplifying the digital imaging signal output from the first ADC unit 401 and the second amplifier for amplifying the digital imaging signal output from the second ADC unit 402 may be provided.

### (Fourth Embodiment)

Next, the fourth embodiment of the present invention will be described. The endoscope system according to the fourth embodiment is different from the configuration of the first chip 21a in the endoscope system 1 according to the above-described second embodiment. Specifically, in the second embodiment, two imaging signals are simultaneously read out from one row and two columns (two photoelectric conversion elements) (two-channel output); however, in the fourth embodiment, four imaging signals are simultaneously read out from two rows and two columns (four photoelectric conversion elements) (4 channel output). Hereinafter, the configuration of the first chip in the endoscope system according to the fourth embodiment will be described.

### [Configuration of First Chip]

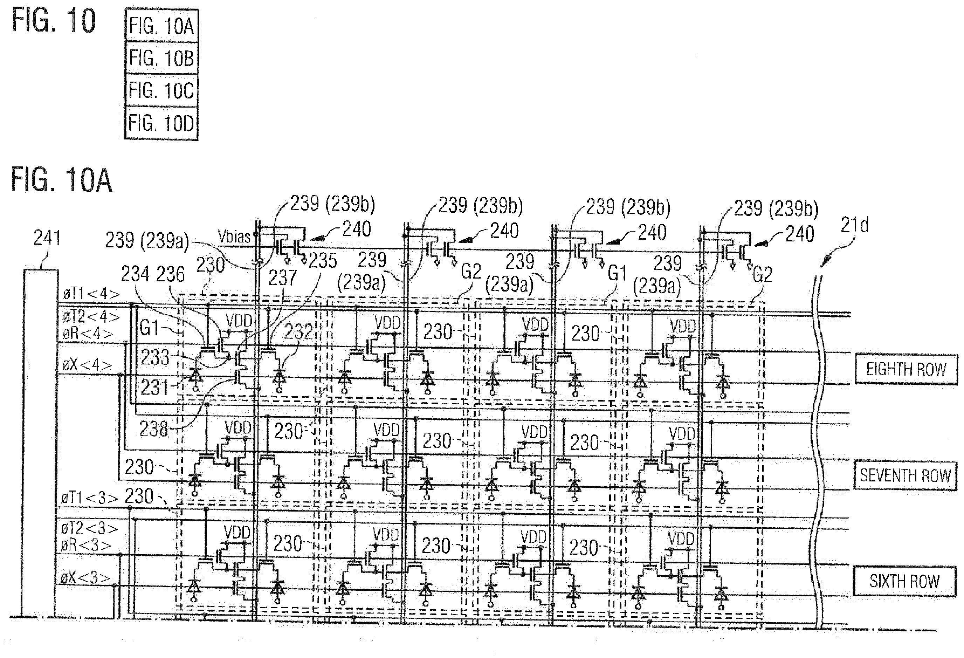

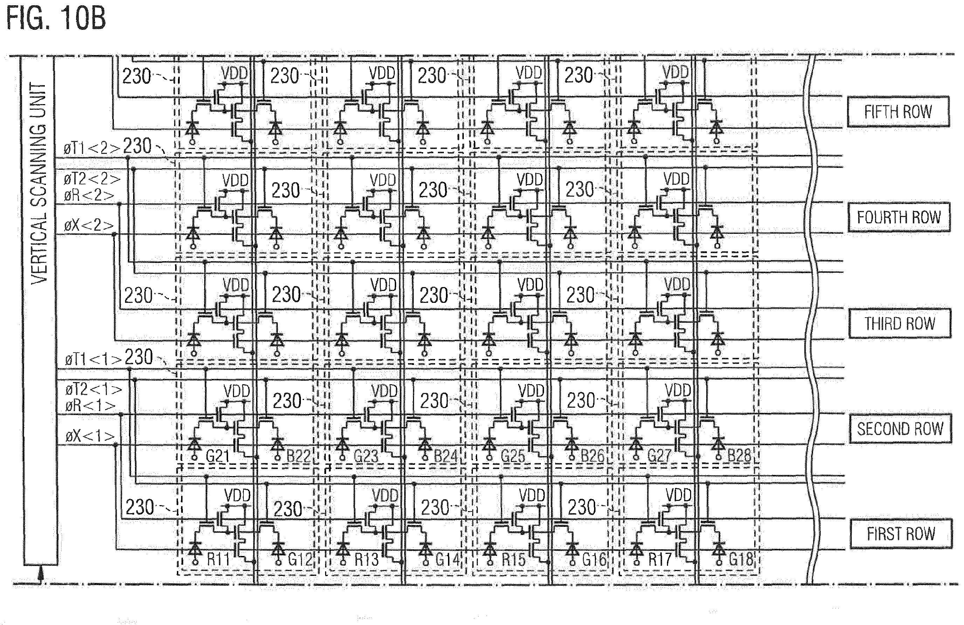

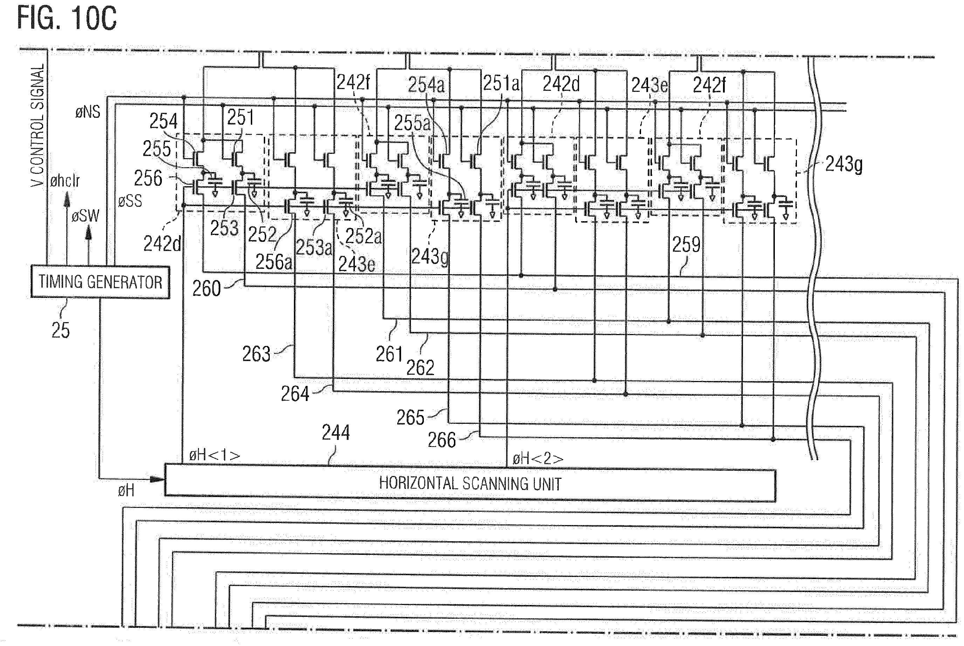

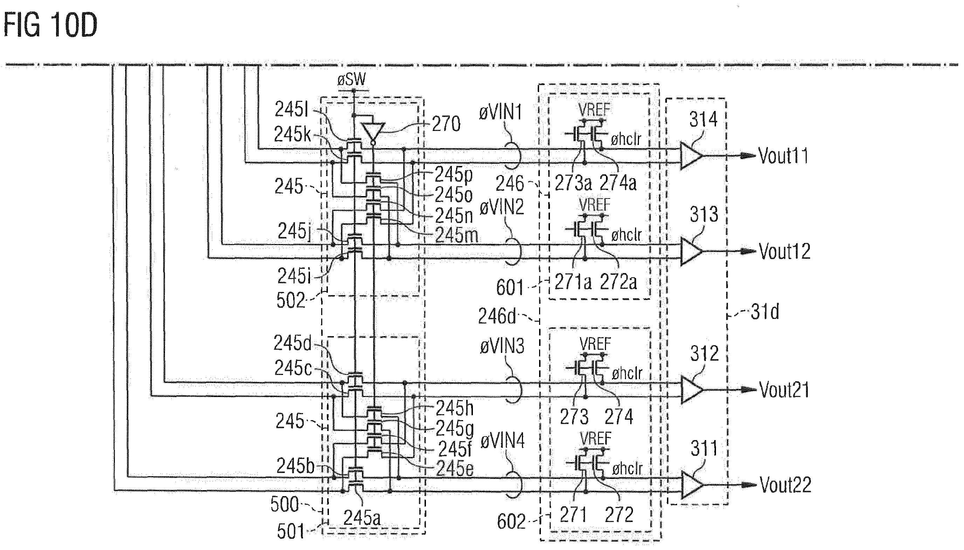

FIG. 10 is a circuit diagram illustrating the configuration of the first chip according to the fourth embodiment of the present invention. In place of the first sample-and-hold unit 242b, the second sample-and-hold unit 243c, the switching unit 245, the horizontal reset unit 246, and the output unit 31 according to the above-described second embodiment, a first chip 21d illustrated in FIG. 10 includes a first sample-and-hold unit 242d, a second sample-and-hold unit 243e, a switching unit 500, a horizontal reset unit 246d, and an output unit 31d. Further, the first chip 21d has a third sample-and-hold unit 242f, a fourth sample-and-hold unit 243g, a fifth horizontal transfer line 263, a sixth horizontal transfer line 264, a seventh horizontal transfer line 265, and an eighth horizontal transfer line 266.

The first sample-and-hold unit 242d (sample-and-hold circuit) samples the noise signal at the time of pixel reset in each unit pixel 230 which outputs the imaging signal via the vertical transfer line 239 of the odd number row and the odd number column, and outputs the sampled noise signal to the first horizontal transfer line 259. Furthermore, the first sample-and-hold unit 242d samples the imaging signal photoelectrically converted by each unit pixel 230 that outputs the imaging signal via the vertical transfer line 239 of the odd number row and the odd number column and outputs the sampled imaging signal to the second horizontal transfer line 260. The first sample-and-hold unit 242d includes the first sampling switch 251, the first sampling unit 252, the first output switch 253, the second sampling switch 254, the second sampling unit 255, and the second output switch 256.

The second sample-and-hold unit 243e samples the noise signal at the time of pixel reset in each unit pixel 230 that outputs the imaging signal via the vertical transfer line 239 of the even number row and the odd number column and outputs the sampled noise signal to the third horizontal transfer line 261. Furthermore, the second sample-and-hold unit 243e samples the imaging signal photoelectrically converted by each unit pixel 230 that outputs the imaging signal via the vertical transfer line 239 of the even number row and the odd number column and outputs the sampled imaging signal to the fourth horizontal transfer line 262. The second sample-and-hold unit 243e includes the first sampling switch 251a, the first sampling unit 252a, the first output switch 253a, the second sampling switch 254a, the second sampling unit 255a, and the second output switch 256a.

The third sample-and-hold unit 242f samples the noise signal at the time of pixel reset in each unit pixel 230 that outputs the imaging signal via the vertical transfer line 239 of the odd number row and the even number column and outputs the sampled noise signal to the fifth horizontal transfer line 263. Furthermore, the third sample-and-hold unit 242f samples the imaging signal photoelectrically converted by each unit pixel 230 that outputs the imaging signal via the vertical transfer line 239 of the odd number row and the even number column and outputs the sampled imaging signal to the sixth horizontal transfer line 264. The third sample-and-hold unit 242f includes the first sampling switch 251, the first sampling unit 252, the first output switch 253, the second sampling switch 254, the second sampling unit 255, and the second output switch 256.

The fourth sample-and-hold unit 243g samples the noise signal at the time of pixel reset in each unit pixel 230 that outputs the imaging signal via the vertical transfer line 239 of the even number row and the even number column and outputs the sampled noise signal to the seventh horizontal transfer line 265. Furthermore, the fourth sample-and-hold unit 243g samples the imaging signal photoelectrically converted by each unit pixel 230 that outputs the imaging signal via the vertical transfer line 239 of the even number row and the even number column and outputs the sampled imaging signal to the eighth horizontal transfer line 266. The fourth sample-and-hold unit 243g includes the first sampling switch 251a, the first sampling unit 252a, the first output switch 253a, the second sampling switch 254a, the second sampling unit 255a, and the second output switch 256a.

On the basis of the drive pulse φSW input from the timing generator 25, the switching unit 500 connects the first horizontal transfer line 259 to the fourth horizontal transfer line 262 to one of the first output amplifier unit 311 and the second output amplifier unit 312 of the output unit 31d to be described later, and connects the fifth horizontal transfer line 263 to the eighth horizontal transfer line 266 to one of the third output amplifier unit 313 and the fourth output amplifier unit 314 of the output unit 31d to be described later. The switching unit 500 includes a first switching unit 501 and a second switching unit 502.

On the basis of the drive pulse φSW supplied from the timing generator 25, the first switching unit 501 connects the first horizontal transfer line 259, the second horizontal transfer line 260, the third horizontal transfer line 261, and the fourth horizontal transfer line 262 to any one of the first output amplifier unit 311 and the second output amplifier unit 312 of the output unit 31d to be described later. The first switching unit 501 includes the first selection switch 245a, the second selection switch 245b, the third selection switch 245c, the fourth selection switch 245d, the fifth selection switch 245e, the sixth selection switch 245f, the seventh selection switch 245g, and the eighth selection switch 245h.

On the basis of the drive pulse φSW supplied from the timing generator 25, the second switching unit 502 connects the fifth horizontal transfer line 263, the sixth horizontal transfer line 264, the seventh horizontal transfer line 265, and the eighth horizontal transfer line 266 to any one of the third output amplifier unit 313 and the fourth output amplifier unit 314 of the output unit 31d to be described later. The second switching unit 502 has the same configuration as the first switching unit 501 and includes a first selection switch 245i, a second selection switch 245j, a third selection switch 245k, a fourth selection switch 2451, a fifth selection switch 245m, a sixth selection switch 245n, a seventh selection switch 245o, an eighth selection switch 245p, and the inversion element 270.

On the basis of the drive pulse φhclr input from the timing generator 25, the horizontal reset unit 246d resets each of the first horizontal transfer line 259 to the eighth horizontal transfer line 266. The horizontal reset unit 246d includes a first horizontal reset unit 601 and a second horizontal reset unit 602.

The first horizontal reset unit 601 includes a first horizontal reset transistor 271, a second horizontal reset transistor 272, a third horizontal reset transistor 273, and a fourth horizontal reset transistor 274.

The second horizontal reset unit 602 has the same configuration as the first horizontal reset unit 601, and includes a first horizontal reset transistor 271a, a second horizontal reset transistor 272a, a third horizontal reset transistor 273a, and a fourth horizontal reset transistor 274a.

The first horizontal reset transistor 271a has one end connected to a reference voltage VREF, the other end connected to the fifth horizontal transfer line 263, and a gate connected to a signal line into which the drive pulse φhclr is input from the timing generator 25. When the drive pulse φhclr is input to the gate of the first horizontal reset transistor 271a from the timing generator 25, the first horizontal reset transistor 271a is turned ON to reset the fifth horizontal transfer line 263.

The second horizontal reset transistor 272a has one end connected to a reference voltage VREF, the other end connected to the sixth horizontal transfer line 264, and a gate connected to a signal line into which the drive pulse φhclr is input from the timing generator 25. When the drive pulse φhclr is input to the gate of the second horizontal reset transistor 272a from the timing generator 25, the second horizontal reset transistor 272a is turned ON to reset the sixth horizontal transfer line 264.

The third horizontal reset transistor 273a has one end connected to a reference voltage VREF, the other end connected to the seventh horizontal transfer line 265, and a gate connected to a signal line into which the drive pulse φhclr is input from the timing generator 25. When the drive pulse φhclr is input to the gate of the third horizontal reset transistor 273a from the timing generator 25, the third horizontal reset transistor 273a is turned ON to reset the seventh horizontal transfer line 265.

The fourth horizontal reset transistor 274a has one end connected to the reference voltage VREF, the other end connected to the eighth horizontal transfer line 266, and a gate connected to a signal line into which the drive pulse φhclr is input from the timing generator 25. When the drive pulse φhclr is input to the gate of the fourth horizontal reset transistor 274a from the timing generator 25, the fourth horizontal reset transistor 274a is turned ON to reset the eighth horizontal transfer line 266.

The output unit 31d takes the difference between the noise signal and the imaging signal transferred from each of the first horizontal transfer line 259 to the eighth horizontal transfer line 266 via the switching unit 500, thereby outputting the imaging signal in which a noise has been removed, to the outside. The output unit 31d includes the first output amplifier unit 311, the second output amplifier unit 312, the third output amplifier unit 313, and the fourth output amplifier unit 314.

The third output amplifier unit 313 is configured using a differential amplifier. The third output amplifier unit 313 takes the difference between the imaging signal transferred from the fifth horizontal transfer line 263 via the switching unit 500 and the noise signal transferred from the sixth horizontal transfer line 264, thereby outputting the imaging signal in which a noise has been removed, to the outside (Vout12).

The fourth output amplifier unit 314 is configured using a differential amplifier, and takes the difference between the imaging signal of the even number column transferred from the seventh horizontal transfer line 265 and the noise signal of the even number column transferred from the eighth horizontal transfer line 266, thereby outputting the imaging signal of the even number column from which noise has been removed, to the outside (Vout11).

The first chip 21d configured in this way performs the same operation as in the above-described first embodiment, thereby simultaneously outputting the imaging signals of two rows and two columns. In this case, the timing generator 25 controls the on/off control of the drive pulse φSW to output imaging signals of the same spectrum (same color filter) from the first output amplifier unit 311 to the fourth output amplifier unit 314. For example, the first output amplifier unit 311 outputs the imaging signal corresponding to the R pixel, the second output amplifier unit 312 outputs the imaging signal corresponding to the G pixel, the third output amplifier unit 313 outputs the imaging signal corresponding to the B pixel, and the fourth output amplifier unit 314 outputs the imaging signal corresponding to G pixels.

According to the fourth embodiment of the present invention described above, the switching unit 500 is controlled to cause the first output amplifier unit 311 to output the imaging signal corresponding to the R pixel, cause the second output amplifier unit 312 to output the imaging signal corresponding to the G pixel, cause the third output amplifier unit 313 to output the imaging signal corresponding to the B pixel, and cause the fourth output amplifier unit 314 to output the imaging signal corresponding to the G pixel. Therefore, in comparison with the case where the imaging signal from the photoelectric conversion element 231 and the photoelectric conversion element 232 each of which is provided with the filter of the same color are processed by one signal processing circuit, it is possible to prevent variations among channels.

### (Other Embodiments)

In the above embodiments, the endoscope configured to be inserted into the subject is employed. Alternatively, a capsule endoscope or an imaging device for imaging a subject may also be employed, for example.

Although the vertical transfer line (first transfer line) shares two pixels in the above embodiments, a vertical transfer line for sharing four pixels or eight pixels, for example, may also be employed. In this case, output amplifiers may be provided in line with the number of shared pixels. Specifically, if one vertical transfer line shares four pixels, four output amplifiers may be provided (four output channels may be provided).

Although a plurality of pixels is shared in the row direction in the above embodiments, a plurality of pixels may be shared in the column direction, for example.

In the description of the timing chart in this specification, before and after the processing between the steps is clearly indicated by using expressions such as "first", "after", and "followed by". However, the order of processing required for implementing the present invention is not uniquely determined by these expressions. That is, the order of processing in the timing charts described in this specification can be changed within a range without inconsistency.

As described above, the present invention may include various embodiments not described here, and it is possible to make various design changes and the like within the scope of the technical idea specified by the claims. Reference Signs List

1 ENDOSCOPE SYSTEM
2 ENDOSCOPE
3 TRANSMISSION CABLE
4 OPERATING UNIT
5 CONNECTOR UNIT
6 PROCESSOR
7 DISPLAY DEVICE
8 LIGHT SOURCE DEVICE
20 IMAGING UNIT
21, 21A, 21B, 21C, 21D FIRST CHIP
22 SECOND CHIP
23 LIGHT RECEIVING UNIT
24 READING UNIT
25 TIMING GENERATOR
26 COLOR FILTER
27 BUFFER
31, 31D, 400 OUTPUT UNIT
51 AFE UNIT
52 A/D CONVERTER
53 IMAGING SIGNAL PROCESSING UNIT
54 DRIVE PULSE GENERATOR
55 POWER SUPPLY VOLTAGE GENERATOR
61 POWER SUPPLY UNIT
62 IMAGE SIGNAL PROCESSING UNIT
63 CLOCK GENERATOR
64 RECORDING UNIT
65 INPUT UNIT
66 PROCESSOR CONTROLLER
100 INSERTION PORTION
101 DISTAL END
102 PROXIMAL END
230 UNIT PIXEL
231, 232 PHOTOELECTRIC CONVERSION ELEMENT
233 CHARGE CONVERTER
234, 235 TRANSFER TRANSISTOR
236 CHARGE CONVERSION RESET UNIT
237 PIXEL SOURCE FOLLOWER TRANSISTOR
238 PIXEL OUTPUT SWITCH
239 VERTICAL TRANSFER LINE
240 CONSTANT CURRENT SOURCE
241 VERTICAL SCANNING UNIT
242, 242B, 242D FIRST SAMPLE-AND-HOLD UNIT
242F THIRD SAMPLE-AND-HOLD UNIT
243, 243C, 243E SECOND SAMPLE-AND-HOLD UNIT
243G FOURTH SAMPLE-AND-HOLD UNIT
244 HORIZONTAL SCANNING UNIT
245, 600, 610 SWITCHING UNIT
245A FIRST SELECTION SWITCH
245B SECOND SELECTION SWITCH
245C, 2451 THIRD SELECTION SWITCH
245D, 245J FOURTH SELECTION SWITCH
245E FIFTH SELECTION SWITCH
245F SIXTH SELECTION SWITCH
245G SEVENTH SELECTION SWITCH
245H EIGHTH SELECTION SWITCH
246, 246D HORIZONTAL RESET UNIT
251, 251A, 251B, 251C FIRST SAMPLING SWITCH
252, 252A, 252B, 252C FIRST SAMPLING UNIT
253, 253A, 253B, 253C FIRST OUTPUT SWITCH
254, 254A, 254B, 254C SECOND SAMPLING SWITCH
255, 255A, 255B, 255C SECOND SAMPLING UNIT
256, 256A, 256B, 256C SECOND OUTPUT SWITCH
259 FIRST HORIZONTAL TRANSFER LINE
260 SECOND HORIZONTAL TRANSFER LINE
261 THIRD HORIZONTAL TRANSFER LINE
262 FOURTH HORIZONTAL TRANSFER LINE
263 FIFTH HORIZONTAL TRANSFER LINE
264 SIXTH HORIZONTAL TRANSFER LINE
265 SEVENTH HORIZONTAL TRANSFER LINE
266 EIGHTH HORIZONTAL TRANSFER LINE
270 INVERSION ELEMENT
271, 271A FIRST HORIZONTAL RESET TRANSISTOR
272, 272A SECOND HORIZONTAL RESET TRANSISTOR
273, 273A THIRD HORIZONTAL RESET TRANSISTOR
274, 274A FOURTH HORIZONTAL RESET TRANSISTOR
311 FIRST OUTPUT AMPLIFIER UNIT
312 SECOND OUTPUT AMPLIFIER UNIT
313 THIRD OUTPUT AMPLIFIER UNIT
314 FOURTH OUTPUT AMPLIFIER UNIT
401 FIRST ADC UNIT
402 SECOND ADC UNIT
500, 600 SWITCHING UNIT
501 FIRST SWITCHING UNIT
502 SECOND SWITCHING UNIT
601 FIRST HORIZONTAL RESET UNIT
602 SECOND HORIZONTAL RESET UNIT
900 CDS CIRCUIT
901 CLAMP CAPACITOR
902 CLAMP SWITCH
903 BUFFER AMPLIFIER
C1 CAPACITOR
G1, G2 PIXEL UNIT

## Claims

1. An image sensor comprising:
a light receiving unit comprising: a plurality of unit pixels arranged in a two-dimensional matrix form, each of the plurality of unit pixels having a plurality of photoelectric converters for converting received light into imaging signals and outputting the imaging signals; and filters having different transmission spectra and disposed on light receiving surfaces of the plurality of photoelectric converters adjacent in a row direction;
a plurality of first transfer lines each sharing a predetermined number of pixels in the row direction and configured to transfer the imaging signals;
a constant current source provided for each of the plurality of first transfer lines and configured to transfer the imaging signals output from the plurality of unit pixels to each of the plurality of first transfer lines;
a plurality of sample-and-hold units provided for the plurality of first transfer lines and configured to sample the imaging signals;
a plurality of second transfer line configured to transfer the imaging signals sampled by the plurality of sample-and-hold units;
a plurality of signal processing units provided corresponding to the number of the plurality of second transfer lines, and configured to perform signal processing on the imaging signals transferred from the plurality of second transfer lines and output the imaging signals to outside;
a switching unit provided between the plurality of second transfer lines and the plurality of signal processing units and configured to switch a connection between the plurality of second transfer lines and the plurality of signal processing units;
a vertical scanning unit configured to output the imaging signals from the plurality of unit pixels to the plurality of sample-and-hold units via the plurality of first transfer lines; and
a control unit configured to cause the vertical scanning unit to:
switch between the plurality of signal processing units with respect to transfer destination of the imaging signals transferred from the plurality of second transfer lines to the switching unit, based on types of the filters disposed on the light receiving surfaces of the plurality of photoelectric converters from which the imaging signals are output; and
output the imaging signals from a single row of the plurality of unit pixels to the plurality of sample-and-hold units, in a predetermined number of times during one horizontal scanning period by dividing the plurality of unit pixels into multiple pixel units each time the imaging signals are output so as to output the imaging signals from the plurality of photoelectric converters having the light receiving surfaces on which the filters of different types are disposed in each of the multiple pixel units.

2. The image sensor according to claim 1, wherein
the control unit is configured to cause the switching unit to switch the transfer destination of the imaging signals transferred from the plurality of second transfer lines so as to transfer the imaging signals output from the plurality of photoelectric converters having the light receiving surfaces on which the filters of a same type are disposed, to a same signal processing unit of the plurality of signal processing units.

3. The image sensor according to claim 1 or 2, wherein each of the plurality of unit pixels comprises:
a charge voltage converter;
a plurality of charge transfer units configured to transfer charges from the plurality of photoelectric converters to the charge voltage converter; and
an output unit configured to output the imaging signals after voltage conversion by the charge voltage converter, wherein
the number of the plurality of charge transfer units in each row of the plurality of unit pixels is two,
each of the two charge transfer units is connected to a first signal line or a second signal line to input a command signal for outputting the imaging signals at different points in time,
the number of the multiple pixel units is two,
a type of one of the filters disposed on one of the plurality of photoelectric converters to output the imaging signals from a first one of the two charge transfer units connected to the first signal line in a first one of the two pixel units is different from a type of another one of the filters disposed on another one of the plurality of photoelectric converters to output the imaging signals from the first one of the two charge transfer units connected to the first signal line in a second one of the two pixel units, and
a type of one of the filters disposed on one of the plurality of photoelectric converters to output the imaging signals from a second one of the two charge transfer units connected to the second signal line in the first one of the two pixel units is different from a type of another one of the filters disposed on another one of the plurality of photoelectric converters to output the imaging signals from the second one of the two charge transfer units connected to the second signal line in the second one of the two pixel units.

4. The image sensor according to any one of claims 1 to 3, wherein
each of the filters disposed on the light receiving surfaces of the plurality of photoelectric converters is one of a red filter, a green filter, and a blue filter,
the number of the plurality of signal processing units is two, and
one of the two signal processing units is configured to perform the signal processing only on the imaging signals output from a photoelectric converter on which the green filter is disposed, among the plurality of photoelectric converters.

5. An endoscope comprising the image sensor according to any one of claims 1 to 4 on a distal end of an insertion portion configured to be inserted into a subject.
